# EUROPEAN PATENT SPECIFICATION

(11) **EP 1 266 005 B1**
(45) Date of publication and mention of the grant of the patent: **07.03.2007**
(21) Application number: 01925421.8
(22) Date of filing: 12.03.2001
(51) Int. Cl.: C12N 15/12, C07K 14/72, C12N 5/10, C12N 1/21, C12Q 1/68

(54) **REGULATION OF HUMAN LIPOXIN A4 RECEPTOR-LIKE PROTEIN**
REGULATION DES MENSCHLICHES LIPOXIN A4 REZEPTOR-ÄHNLICHES PROTEIN
REGULATION DE LA PROTEINE DE TYPE DU RECEPTEUR DE LA LIPOXINE A4 HUMAINE

(30) Priority: 14.03.2000 US 189037 P
(43) Date of publication of application: 18.12.2002
(73) Proprietor: Bayer HealthCare AG, 51368 Leverkusen (DE)
(72) Inventor: RAMAKRISHNAN, Shyam, Brighton, MA 02135 (US)
(86) International application number: PCT/EP2001/002729
(87) International publication number: WO 2001/068839

(56) References cited:
- EP-A- 1 094 075
- WO-A-00/22131
- WO-A-00/26339
- WO-A-00/31258
- WO-A-01/36473
- WO-A-01/42288
- DE-A- 19 930 512
- DATABASE EMBL [Online] ACC NO: AC005849, 23 October 1998 (1998-10-23) retrieved from EBI XP002183865
- J F MADDOX ET AL.: "Lipoxin A4 Stable Analogs are potent Mimetics that stimulate human monocytes and THP-1 cells via a G-protein-coupled Lipoxin A4 Receptor" J BIOL CHEM, vol. 272, no. 11, 14 March 1997 (1997-03-14), pages 6972-6978, XP002183863

## Description

### TECHNICAL FIELD OF THE INVENTION

The invention relates to the area of G-protein coupled receptors. More particularly, it relates to the area of lipoxin A₄ receptor-like proteins and their use in drug screening in the field of inflammatory diseases.

### BACKGROUND OF THE INVENTION

### G-Protein Coupled Receptors

Many medically significant biological processes are mediated by signal transduction pathways that involve G-proteins (Lefkowitz, *Nature 351,* 353-354, 1991). The family of G-protein coupled receptors (GPCR) includes receptors for hormones, neurotransmitters, growth factors, and viruses. Specific examples of GPCRs include receptors for such diverse agents as dopamine, calcitonin, adrenergic hormones, endothelin, cAMP, adenosine, acetylcholine, serotonin, histamine, thrombin, kinin, follicle stimulating hormone, opsins, endothelial differentiation gene-1, rhodopsins, odorants, cytomegalovirus, G-proteins themselves, effector proteins such as phospholipase C, adenyl cyclase, and phosphodiesterase, and actuator proteins such as protein kinase A and protein kinase C.

GPCRs possess seven conserved membrane-spanning domains connecting at least eight divergent hydrophilic loops. GPCRs (also known as 7TM receptors) have been characterized as including these seven conserved hydrophobic stretches of about 20 to 30 amino acids, connecting at least eight divergent hydrophilic loops. Most GPCRs have single conserved cysteine residues in each of the first two extracellular loops, which form disulfide bonds that are believed to stabilize functional protein structure. The seven transmembrane regions are designated as TM1, TM2, TM3, TM4, TM5, TM6, and TM7. TM3 has been implicated in signal transduction.

Phosphorylation and lipidation (palmitylation or farnesylation) of cysteine residues can influence signal transduction of some GPCRs. Most GPCRs contain potential phosphorylation sites within the third cytoplasmic loop and/or the carboxy terminus. For several GPCRs, such as the β-adrenergic receptor, phosphorylation by protein kinase A and/or specific receptor kinases mediates receptor desensitization.

For some receptors, the ligand binding sites of GPCRs are believed to comprise hydrophilic sockets formed by several GPCR transmembrane domains. The hydrophilic sockets are surrounded by hydrophobic residues of the GPCRs. The hydrophilic side of each GPCR transmembrane helix is postulated to face inward and form a polar ligand binding site. TM3 has been implicated in several GPCRs as having a ligand binding site, such as the TM3 aspartate residue. TM5 serines, a TM6 asparagine, and TM6 or TM7 phenylalanines or tyrosines also are implicated in ligand binding.

GPCRs are coupled inside the cell by heterotrimeric G-proteins to various intracellular enzymes, ion channels, and transporters (see Johnson *et al., Endoc. Rev. 10,* 317-331, 1989). Different G-protein alpha-subunits preferentially stimulate particular effectors to modulate various biological functions in a cell. Phosphorylation of cytoplasmic residues of GPCRs is an important mechanism for the regulation of some GPCRs. For example, in one form of signal transduction, the effect of hormone binding is the activation of the enzyme, adenylate cyclase, inside the cell. Enzyme activation by hormones is dependent on the presence of the nucleotide GTP. GTP also influences hormone binding. A G-protein connects the hormone receptor to adenylate cyclase. G-protein exchanges GTP for bound GDP when activated by a hormone receptor. The GTP-carrying form then binds to activated adenylate cyclase. Hydrolysis of GTP to GDP, catalyzed by the G-protein itself, returns the G-protein to its basal, inactive form. Thus, the G-protein serves a dual role, as an intermediate that relays the signal from receptor to effector, and as a clock that controls the duration of the signal.

Over the past 15 years, nearly 350 therapeutic agents targeting 7TM receptors have been successfully introduced onto the market. This indicates that these receptors have an established, proven history as therapeutic targets. Clearly, there is a need for identification and characterization of further receptors which can play a role in preventing, ameliorating, or correcting dysfunctions or diseases including, but not limited to, infections such as bacterial, fungal, protozoan, and viral infections, particularly those caused by HIV viruses, pain, cancers, anorexia, bulimia, asthma, Parkinson's diseases, acute heart failure, hypotension, hypertension, urinary retention, osteoporosis, angina pectoris, myocardial infarction, ulcers, asthma, allergies, benign prostatic hypertrophy, and psychotic and neurological disorders, including anxiety, schizophrenia, manic depression, delirium, dementia, several mental retardation, and dyskinesias, such as Huntington's disease and Tourett's syndrome.

### Lipoxin A₄

Lipoxins (lipoxygenase interaction products) are a novel series of arachidonic acid derived metabolites (Serhan *et al., Biochem. Biophys. Res. Commun. 18*, 943-949 (1984); Serhan *et al., Proc. Natl. Acad. Sci. USA ,* 5335-5339, 1984). The distinguishing feature of a lipoxin is the presence of a trihydroxy conjugated tetraene structure. At least two biologically active lipoxins have been characterized: lipoxin A₄ [(5S,6R,15S)-5,6,15-trihydroxy-7,9,13-trans-11-cis-eicosa-tetraenoic acid] and lipoxin B₄ [(5S,14R,15S)-5,14,15-trihydroxy-6,10,12-trans-8-cis-eicosatetraenoic acid] (Serhan. *et al., J. Biol. Chem. 261,* 16340-16345; Serhan *et al., Proc. Natl. Acad. Sci. USA 83*, 1983-1987, 1986).

Lipoxin A₄ has been shown to contract pulmonary smooth muscle (guinea pig lung), but not guinea pig ileum or trachea, and to relax (dilate) vascular smooth muscle at concentrations of less than 1 µM (Dahlen *et al., Acta Physiol. Scand 130,* 643-647, 1987). Topical administration of lipoxin A₄ to the hamster cheek pouch induces a pronounced arteriolar dilation, but does not change venular diameters (Dahlen *et al.,* in ADV. EXPER. MED. BIOL. 229, Chapter 9, pp. 107-130, 1988). Lipoxin A₄ has also been shown to induce neutrophils to generate superoxide radicals, release elastase, and promote chemotaxis by leukocytes (Serhan *et al.,* in PROSTAGLANDINS, LEUKOTRIENES AND LIPOXINS, J. M. Bailey, ed., Plenum, N.Y., pp. 3-16, 1985).

It has been suggested to use lipoxin A₄ to induce the inflammatory response of neutrophils so as to provide an experimental model to evaluate the efficacy of compounds such as lipoxin B₄ derivatives in preventing this response (Samuelsson *et al.,* U.S. Patent 4,560,514). It has also been suggested that lipoxin A₄ may exert some of its biological effects by binding to the lipoxin D₄ receptor (Jacques *et al., Br. J. Pharmacol. 95,* 562-568, 1988) and that lipoxin A₄ and LTD₄ may even share a common receptor (Lefer *et al., Proc. Natl. Acad Sci. USA 85*, 8340-8344, 1988).

Studies in human neutrophils have established an inverse relation between the generation of lipoxins and leukotrienes following exposure of these cells to 15-HETE and the calcium ionophore A23187 (Serhan, in ADVANCES IN PROSTAGLANDIN, THROMBOXANE AND LEUKOTRIENE RESEARCH, B. Samuelsson *et al.,* eds., Raven Press, N.Y., Volume 18). In addition, recent results indicate that mesangial cells can generate lipoxins from exogenous sources of LTA₄ (Garrick *et al.,* Kidney Int. Proceedings 21st Annual Meeting of the American Society of Nephrology, Vol. 25, p. 292, 1989), which may be provided by activated leukocytes (*e.g.*, during transcellular metabolism). Thus, the local levels of these compounds may be elevated following the infiltration of leukocytes into the glomerulus.

### SUMMARY OF THE INVENTION

An embodiment of the invention is a method of screening for agents which can regulate the activity of a lipoxin A₄ receptor-like polypeptide. A test compound is contacted with a lipoxin A₄ receptor-like polypeptide comprising an amino acid sequence selected from the group consisting of amino acid sequences which are at least 90% identical to the amino acid sequence shown in SEQ ID NO. 2 and the amino acid sequence shown in SEQ ID NO. 2. Binding of the test compound to the polypeptide is detected. A test compound which binds to the polypeptide is thereby identified as a potential agent for regulating activity of a lipoxin A₄ receptor-like polypeptide useful in the treatment of a disease associated with inflammatory processes.

Another embodiment of the invention is a method of screening for agents useful in the treatment of a disease associated with inflammatory processes which reduce the activity of a lipoxin A₄ receptor-like polypeptide. A test compound is contacted or incubated with a biological cell, wherein the biological is a T-cell and/or has been transformed with a polynucleotide wherein the polynucleotide comprises a nucleotide sequence selected from the group consisting of nucleotide sequences which hybridize under stringent conditions to the nucleotide sequence shown in SEQ ID NO. 1 and the nucleotide sequence shown in SEQ ID NO. 1. Binding of the test compound to the polynucleotide is detected. A test compound which binds to the polynucleotide is identified as a potential agent for reducing the activity of a lipoxin A₄ receptor-like polypeptide. The agent can work by decreasing the amount of the lipoxin A₄ receptor-like through interacting with the lipoxin A₄ receptor-like mRNA.

### BRIEF DESCRIPTION OF THE DRAWINGS

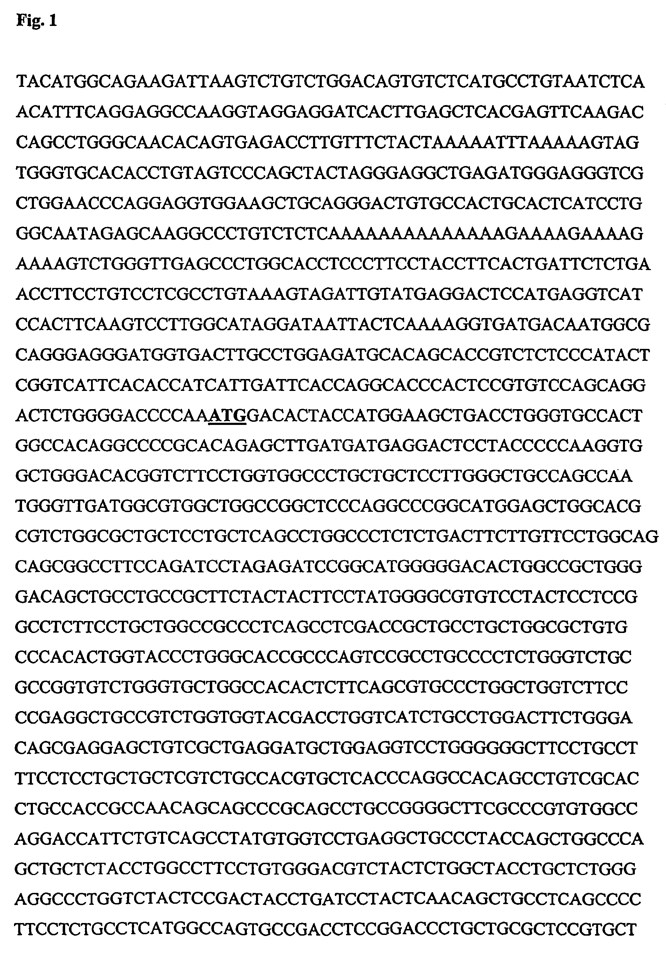

Fig. 1 shows the DNA-sequence encoding a lipoxin A₄ receptor-like polypeptide.

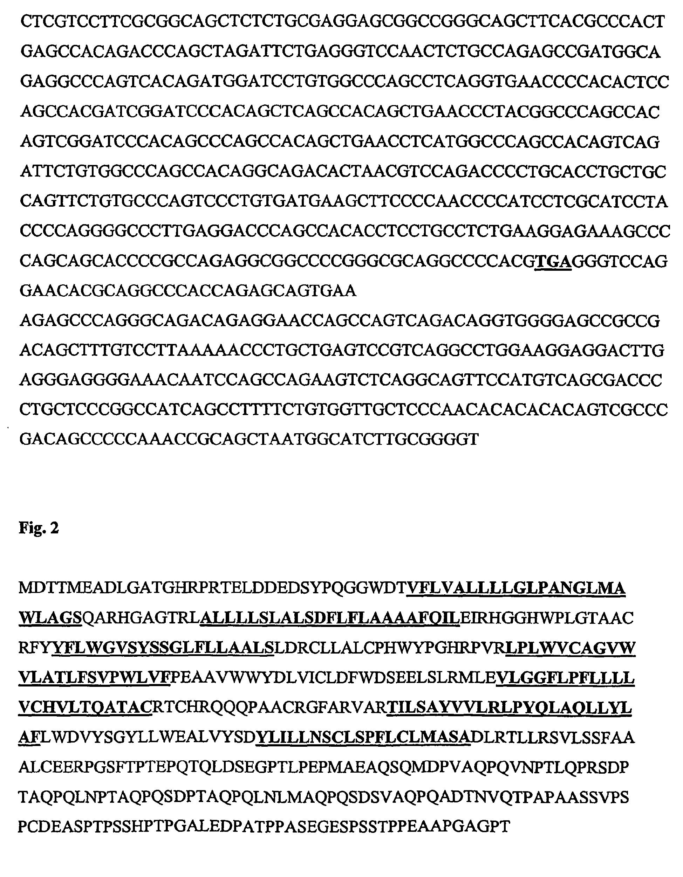

Fig. 2 shows the amino acid sequence of a lipoxin A₄ receptor-like polypeptide.

### DETAILED DESCRIPTION OF THE INVENTION

The invention relates to the use as defined by the claims of an isolated polynucleotide encoding a lipoxin A₄ receptor-like polypeptide and being selected from the group consisting of:
a) a polynucleotide comprising the sequence of SEQ ID NO. 1;
b) a polynucleotide which hybridizes under stringent conditions to a polynucleotide specified in (a);
c) a polynucleotide, comprising a polynucleotide as defined under a) or b).

Human lipoxin A₄ receptor-like polypeptide also can be used to screen for lipoxin A₄ receptor-like agonists and antagonits useful in the treatment of a disease associated with inflammatory processes.

The amino acid sequence of human lipoxin A₄ receptor-like polypeptide useful for the invention as defined by the claims is shown in SEQ ID NO. 2 or is at least 90% identical to SEQ ID NO: 2. A nucleotide sequence which encodes SEQ ID NO. 2 is shown in SEQ ID NO. 1, with the start and stop codons indicated.

### Lipoxin A₄ Receptor-Like Polypeptides

Lipoxin A₄ receptor-like polypeptides useful for the invention as defined by the claims comprise the amino acid sequence shown in SEQ ID NO. 2, or are at least 90% identical to SEQ ID NO: 2. A lipoxin A₄ receptor-like polypeptide of the invention therefore can be a portion of a lipoxin A₄ receptor-like molecule, a full-length lipoxin A₄ receptor-like molecule, or a fusion protein comprising all or a portion of a lipoxin A₄ receptor-like molecule.

Preferably, a lipoxin A₄ receptor-like polypeptide binds lipoxin A₄ or a lipoxin A₄ analog. Binding can be determined as described, for example, in the specific examples below.

### Biologically Active Variants

Lipoxin ₄A receptor-like polypeptide variants which are biologically active, *i.e.,* retain the ability to bind lipoxin A₄ or a lipoxin A₄ analog, and/or which mediate a biological effect, such- as cyclic AMP formation, mobilization of intracellular calcium or phosphinositide metabolism, also are lipoxin A₄ receptor-like polypeptides. Percent identity between a putative lipoxin A₄ receptor-like polypeptide variant and an amino acid sequence of SEQ ID NO. 2 is determined using the Blast2 alignment program.

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a lipoxin A₄ receptor-like polypeptide can be found using computer programs well known in the art, such as DNASTAR software. Whether an amino acid change results in a biologically active lipoxin A₄ receptor-like polypeptide can readily be determined by assaying for lipoxin A₄ receptor-like polypeptide activity, as described, for example, in the specific examples below.

### Fusion Proteins

Fusion proteins are useful for generating antibodies against lipoxin A₄ receptor-like polypeptide amino acid sequences and for use in various assay systems. For example, fusion proteins can be used to identify proteins which interact with portions of a lipoxin A₄ receptor-like polypeptide. Protein affinity chromatography or library-based assays for protein-protein interactions, such as the yeast two-hybrid or phage display systems, can be used for this purpose. Such methods are well known in the art and also can be used as drug screens.

A lipoxin A₄ receptor-like polypeptide fusion protein comprises two polypeptide segments fused together by means of a peptide bond. Contiguous amino acids for use in a fusion protein can be selected from the amino acid sequence shown in SEQ ID NO. 2 or from a biologically active variant of those sequences, such as those described above. The first polypeptide segment also can comprise full-length lipoxin A₄ receptor-like polypeptide.

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located between the lipoxin A₄ receptor-like polypeptide-encoding sequence and the heterologous protein sequence, so that the lipoxin A₄ receptor-like polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from SEQ ID NO. 1 in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KITS).

### Identification of Species Homologs

Species homologs of human lipoxin A₄ receptor-like polypeptide can be obtained using lipoxin A₄ receptor-like polypeptide polynucleotides (described below) to make suitable probes or primers for screening cDNA expression libraries from other species, such as mice, monkeys, or yeast, identifying cDNAs which encode homologs of lipoxin A₄ receptor-like polypeptide, and expressing the cDNAs as is known in the art.

### Lipoxin A₄ Receptor-Like Polynucleotides

A lipoxin A₄ receptor-like polynucleotide can be single- or double-stranded and comprises a coding sequence or the complement of a coding sequence for a lipoxin A₄ receptor-like polypeptide. A coding sequence for human lipoxin A₄ receptor-like polypeptide is shown in SEQ ID NO. 1.

Degenerate nucleotide sequences encoding human lipoxin A₄ receptor-like polypeptides, as well as homologous nucleotide sequences which are at least about 50, preferably about 75, 90, 96, or 98% identical to the nucleotide sequence shown in SEQ ID NO. 1 also are lipoxin A₄ receptor-like polynucleotides. Percent sequence identity between the sequences of two polynucleotides is determined using computer programs such as ALIGN which employ the FASTA algorithm, using an affine gap search with a gap open penalty of -12 and a gap extension penalty of -2. Complementary DNA (cDNA) molecules, species homologs, and variants of lipoxin A₄ receptor-like polynucleotides which encode biologically active lipoxin A₄ receptor-like polypeptides also are lipoxin A₄ receptor-like polynucleotides.

### Identification of Variants and Homologs of Lipoxin A₄ Receptor-Like Polypeptide Polynucleotides

Variants and homologs of the lipoxin A₄ receptor-like polynucleotides described above also are lipoxin A₄ receptor-like polynucleotides. Typically, homologous lipoxin A₄ receptor-like polynucleotide sequences can be identified by hybridization of candidate polynucleotides to known lipoxin A₄ receptor-like polynucleotides under stringent conditions, as is known in the art. For example, using the following wash conditions-2X SSC (0.3 M NaCl, 0.03 M sodium citrate, pH 7.0), 0.1 % SDS, room temperature twice, 30 minutes each; then 2X SSC, 0.1% SDS, 50 C once, 30 minutes; then 2X SSC, room temperature twice, 10 minutes each--homologous sequences can be identified which contain at most about 25-30% basepair mismatches. More preferably, homologous nucleic acid strands contain 15-25% basepair mismatches, even more preferably 5-15% basepair mismatches.

Species homologs of the lipoxin A₄ receptor-like polynucleotides disclosed herein also can be identified by making suitable probes or primers and screening cDNA expression libraries from other species, such as mice, monkeys, or yeast. Human variants of lipoxin A₄ receptor-like polynucleotides can be identified, for example, by screening human cDNA expression libraries. It is well known that the Tₘ of a double-stranded DNA decreases by 1-1.5°C with every 1% decrease in homology (Bonner *et al., J. Mol. Biol. 81,* 123 (1973). Variants of human lipoxin A₄ receptor like polynucleotides or lipoxin A₄ receptor-like polynucleotides of other species can therefore be identified by hybridizing a putative homologous lipoxin A₄ receptor-like polynucleotide with a polynucleotide having a nucleotide sequence of SEQ ID NO. 1 or the complement thereof to form a test hybrid. The melting temperature of the test hybrid is compared with the melting temperature of a hybrid comprising transformylase polynucleotides having perfectly complementary nucleotide sequences, and the number or percent of basepair mismatches within the test hybrid is calculated.

Nucleotide sequences which hybridize to transformylase polynucleotides or their complements following stringent hybridization and/or wash conditions are also lipoxin A₄ receptor-like polynucleotides. Stringent wash conditions are well known and understood in the art and are disclosed, for example, in Sambrook *et al.,* MOLECULAR CLONING: A LABORATORY MANUAL, 2d ed., 1989, at pages 9.50-9.51.

Stringent wash conditions include, for example, 4X SSC at 65°C, or 50% formamide, 4X SSC at 42°C, or 0.5X SSC, 0.1% SDS at 65°C. Highly stringent wash conditions include, for example, 0.2X SSC at 65°C.

### Preparation of Lipoxin A₄ Receptor-Like Polynucleotides

A naturally occurring lipoxin A₄ receptor-like polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated lipoxin A₄ receptor-like polynucleotides. For example, restriction enzymes and probes can be used to isolate polynucleotide fragments which comprises lipoxin A₄ receptor-like nucleotide sequences. Isolated polynucleotides are in preparations which are free or at least 70, 80, or 90% free of other molecules.

Lipoxin A₄ receptor-like cDNA molecules can be made with standard molecular biology techniques, using lipoxin A₄ receptor-like mRNA as a lipoxin A₄ receptor-like. Lipoxin A₄ receptor-like cDNA molecules can thereafter be replicated using molecular biology techniques known in the art and disclosed in manuals such as Sambrook *et al.* (1989). An amplification technique, such as PCR, can be used to obtain additional copies of polynucleotides of the invention, using either human genomic DNA or cDNA as a template.

Alternatively, synthetic chemistry techniques can be used to synthesizes lipoxin A₄ receptor-like polynucleotides. The degeneracy of the genetic code allows alternate nucleotide sequences to be synthesized which will encode a lipoxin A₄ receptor-like polypeptide having, for example, an amino acid sequence shown in SEQ ID NO. 2 or a biologically active variant thereof.

### Obtaining Lipoxin A₄ Receptor-Like Polypeptides

Lipoxin A₄ receptor-like polypeptides can be obtained, for example, by purification from human cells, by expression of lipoxin A₄ receptor-like polynucleotides, or by direct chemical synthesis.

### Protein Purification

Lipoxin A₄ receptor-like polypeptides can be purified from any human cell which expresses the receptor, including any host cell which has been transfected with a lipoxin A₄ receptor-like polypeptide-expressing polynucleotide construct. A purified lipoxin A₄ receptor-like polypeptide is separated from other compounds which normally associate with the lipoxin A₄ receptor-like polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

Lipoxin A₄ receptor-like polypeptide is conveniently isolated as a complex with its associated G protein. A variety of lipoxin A₄ receptor-like analogs are available and can be used as ligands for receptor binding. Biotinylated lipoxin A₄ receptor-like analogs are particularly useful for this purpose. A preparation of purified lipoxin A₄ receptor-like polypeptides is at least 80% pure; preferably, the preparations are 90%, 95%, or 99% pure. Purity of the preparations can be assessed by any means known in the art, such as SDS-polyacrylamide gel electrophoresis.

### Expression of Lipoxin A₄ Receptor-Like Polynucleotides

To express a lipoxin A₄ receptor-like polypeptide, a lipoxin A₄ receptor-like polynucleotide can be inserted into an expression vector which contains the necessary elements for the transcription and translation of the inserted coding sequence.

Methods which are well known to those skilled in the art can be used to construct expression vectors containing sequences encoding lipoxin A₄ receptor-like polypeptides and appropriate transcriptional and translational control elements. These methods include *in vitro* recombinant DNA techniques, synthetic techniques, and *in vivo* genetic recombination. Such techniques are described, for example, in Sambrook *et al.* (1989) and in Ausubel *et al.,* CURRENT PROTOCOLS IN MOLECULAR BIOLOGY, John Wiley & Sons, New York, N.Y, 1989.

A variety of expression vector/host systems can be utilized to contain and express sequences encoding a lipoxin A₄ receptor-like polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (*e.g.*, baculovirus), plant cell systems transformed with virus expression vectors (*e.g.*, cauliflower mosaic virus, CaMV; tobacco mosaic virus, TMV) or with bacterial expression vectors (*e.g.*, Ti or pBR322 plasmids), or animal cell systems.

The control elements or regulatory sequences are those non-translated regions of the vector - enhancers, promoters, 5' and 3' untranslated regions - which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (*e.g.,* heat shock, RUBISCO, and storage protein genes) or from plant viruses (*e.g.,* viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding a lipoxin A₄ receptor-like polypeptide, vectors based on SV40 or EBV can be used with an appropriate selectable marker.

### Bacterial and Yeast Expression Systems

In bacterial systems, a number of expression vectors can be selected depending upon the use intended for the lipoxin A₄ receptor-like polypeptide. For example, when a large quantity of a lipoxin A₄ receptor-like polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the lipoxin A₄ receptor-like polypeptide can be ligated into the vector in frame with sequences for the amino-terminal Met and the subsequent 7 residues of β-galactosidase so that a hybrid protein is produced. pIN vectors (Van Heeke & Schuster, *J. Biol. Chem. 264,* 5503-5509, 1989) or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or Factor Xa protease cleavage sites so that the cloned polypeptide of interest can be released from the GST moiety at will.

In the yeast *Saccharomyces cerevisiae,* a number of vectors containing constitutive or inducible promoters such as alpha factor, alcohol oxidase, and PGH can be used. For reviews, see Ausubel *et al.* (1989) and Grant *et al., Methods Enzymol. 153,* 516-544, 1987.

### Plant and Insect Expression Systems

If plant expression vectors are used, the expression of sequences encoding lipoxin A₄ receptor-like polypeptides can be driven by any of a number of promoters. For example, viral promoters such as the 35S and 19S promoters of CaMV can be used alone or in combination with the omega leader sequence from TMV (Takamatsu *EMBO J. 6,* 307-311, 1987). Alternatively, plant promoters such as the small subunit of RUBISCO or heat shock promoters can be used (Coruzzi *et al., EMBO J. 3,* 1671-1680, 1984; Broglie *et al., Science 224,* 838-843, 1984; Winter *et al., Results Probl. Cell Differ. 17*, 85-105, 1991). These constructs can be introduced into plant cells by direct DNA transformation or by pathogen-mediated transfection. Such techniques are described in a number of generally available reviews (*e.g.,* Hobbs or Murry, in McGRAW HILL YEARBOOK OF SCIENCE AND TECHNOLOGY, McGraw Hill, New York, N.Y., pp. 191-196, 1992).

An insect system also can be used to express a lipoxin A₄ receptor-like polypeptide. For example, in one such system *Autographa californica* nuclear polyhedrosis virus (AcNPV) is used as a vector to express foreign genes in *Spodoptera frugiperda* cells or in *Trichoplusia* larvae. Sequences encoding lipoxin A₄ receptor-like polypeptides can be cloned into a non-essential region of the virus, such as the polyhedrin gene, and placed under control of the polyhedrin promoter. Successful insertion of lipoxin A₄ receptor-like polypeptides will render the polyhedrin gene inactive and produce recombinant virus lacking coat protein. The recombinant viruses can then be used to infect *S. frugiperda cells* or *Trichoplusia* larvae in which lipoxin A₄ receptor-like polypeptides can be expressed (Engelhard *et al., Proc. Nat. Acad Sci. 91,* 3224-3227, 1994).

### Mammalian Expression Systems

A number of viral-based expression systems can be used to express lipoxin A₄ receptor-like polypeptides in mammalian host cells. For example, if an adenovirus is used as an expression vector, sequences encoding lipoxin A₄ receptor-like polypeptides can be ligated into an adenovirus transcription/translation complex comprising the late promoter and tripartite leader sequence. Insertion in a non-essential E1 or E3 region of the viral genome can be used to obtain a viable virus which is capable of expressing a lipoxin A₄ receptor-like polypeptide in infected host cells (Logan & Shenk, *Proc. Natl. Acad Sci. 81,* 3655-3659, 1984). If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (*e.g.*, liposomes, polycationic amino polymers, or vesicles).

Specific initiation signals also can be used to achieve more efficient translation of sequences encoding lipoxin A₄ receptor-like polypeptides. Such signals include the ATG initiation codon and adjacent sequences. In cases where sequences encoding a lipoxin A₄ receptor-like polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used (see Scharf *et al., Results Probl. Cell Differ. 20,* 125-162, 1994).

### Host Cells

A host cell strain can be chosen for its ability to modulate the expression of the inserted sequences or to process the expressed lipoxin A₄ receptor-like polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (*e.g.,* CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express lipoxin A₄ receptor-like polypeptides can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced lipoxin A₄ receptor- like sequences. Resistant clones of stably transformed cells can be proliferated using tissue culture techniques appropriate to the cell type. See, for example, ANIMAL CELL CULTURE, R.I. Freshney, ed., 1986.

Any number of selection systems can be used to recover transformed cell lines.

These include, but are not limited to, the herpes simplex virus thymidine kinase (Wigler *et al., Cell 11,* 223-32, 1977) and adenine phosphoribosyltransferase (Lowy *et al., Cell 22*, 817-23, 1980) genes which can be employed in *tk*⁻ or *aprt⁻* cells, respectively. Also, antimetabolite, antibiotic, or herbicide resistance can be used as the basis for selection. For example, *dhfr* confers resistance to methotrexate (Wigler *et al., Proc. Natl. Acad Sci. 77, 3567-70,* 1980) *npt* confers resistance to the aminoglycosides, neomycin and G-418 (Colbere-Garapin *et al., J. Mol. Biol. 150,* 1-14, 1981), and *als* and *pat* confer resistance to chlorsulfuron and phosphinotricin acetyltransferase, respectively (Murry, 1992, *supra*). Additional selectable genes have been described. For example, *trpB* allows cells to utilize indole in place of tryptophan, or *hisD,* which allows cells to utilize histinol in place of histidine (Hartman & Mulligan, *Proc. Natl. Acad Sci. 85*, 8047-51, 1988). Visible markers such as anthocyanins, β-glucuronidase and its substrate GUS, and luciferase and its substrate luciferin, can be used to identify transformants and to quantify the amount of transient or stable protein expression attributable to a specific vector system (Rhodes *et al., Methods Mol. Biol. 55,* 121-131, 1995).

### Detecting Expression of Lipoxin A₄ Receptor-Like Polypeptides

Although the presence of marker gene expression suggests that the lipoxin A₄ receptor-like polynucleotide is also present, its presence and expression may need to be confirmed. For example, if a sequence encoding a lipoxin A₄ receptor-like polypeptide is inserted within a marker gene sequence, transformed cells containing sequences which encode a lipoxin A₄ receptor-like polypeptide can be identified by the absence of marker gene function. Alternatively, a marker gene can be placed in tandem with a sequence encoding a lipoxin A₄ receptor-like polypeptide under the control of a single promoter. Expression of the marker gene in response to induction or selection usually indicates expression of the lipoxin A₄ receptor-like polynucleotide.

Alternatively, host cells which contain a lipoxin A₄ receptor-like polynucleotide and which express a lipoxin A₄ receptor-like polypeptide can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding a lipoxin A₄ receptor-like polypeptide can be detected by DNA-DNA or DNA-RNA hybridization or amplification using probes or fragments or fragments of polynucleotides encoding a lipoxin A₄ receptor-like polypeptide. Nucleic acid amplification-based assays involve the use of oligonucleotides selected from sequences encoding a lipoxin A₄ receptor-like polypeptide to detect transformants which contain a lipoxin A₄ receptor-like polynucleotide.

A variety of protocols for detecting and measuring the expression of a lipoxin A₄ receptor-like polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a lipoxin A₄ receptor-like polypeptide can be used, or a competitive binding assay can be employed. These and other assays are described in Hampton *et al.,* SEROLOGICAL METHODS: A LABORATORY MANUAL, APS Press, St. Paul, Minn., 1990) and Maddox *et al., J. Exp. Med. 158,* 1211-1216, 1983).

A wide variety of labels and conjugation techniques are known by those skilled in the art and can be used in various nucleic acid and amino acid assays. Means for producing labeled hybridization or PCR probes for detecting sequences related to polynucleotides encoding lipoxin A₄ receptor-like polypeptides include oligo-labeling, nick translation, end-labeling, or PCR amplification using a labeled nucleotide. Alternatively, sequences encoding a lipoxin A₄ receptor-like polypeptide can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

### Expression and Purification of Lipoxin A₄ Receptor-Like Polypeptides

Host cells transformed with nucleotide sequences encoding a lipoxin A₄ receptor-like polypeptide can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing polynucleotides which encode lipoxin A₄ receptor-like polypeptides can be designed to contain signal sequences which direct secretion of soluble lipoxin A₄ receptor-like polypeptides through a prokaryotic or eukaryotic cell membrane or which direct the membrane insertion of membrane-bound lipoxin A₄ receptor-like polypeptide.

As discussed above, other constructions can be used to join a sequence encoding a lipoxin A₄ receptor-like polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the lipoxin A₄ receptor- like polypeptide also can be used to facilitate purification. One such expression vector provides for expression of a fusion protein containing a lipoxin A₄ receptor-like polypeptide and 6 histidine residues preceding a thioredoxin or an enterokinase cleavage site. The histidine residues facilitate purification by IMAC (immobilized metal ion affinity chromatography, as described in Porath *et al., Prot. Exp. Purif. 3,* 263-281, 1992), while the enterokinase cleavage site provides a means for purifying the lipoxin A₄ receptor-like polypeptide from the fusion protein. Vectors which contain fusion proteins are disclosed in Kroll *et al., DNA Cell Biol. 12*, 441-453, 1993.

### Chemical Synthesis

Sequences encoding a lipoxin A₄ receptor-like polypeptide can be synthesized, in whole or in part, using chemical methods well known in the art (see Caruthers *et al., Nucl. Acids Res. Symp. Ser.* 215-223, 1980; Hom *et al. Nucl. Acids Res. Symp. Ser.* 225-232, 1980). Alternatively, a lipoxin A₄ receptor-like polypeptide itself can be produced using chemical methods to synthesize its amino acid sequence, such as by direct peptide synthesis using solid-phase techniques (Merrifield, *J. Am. Chem. Soc. 85,* 2149-2154, 1963; Roberge *et al., Science 269,* 202-204, 1995). Protein synthesis can be performed using manual techniques or by automation. Automated synthesis can be achieved, for example, using Applied Biosystems 431A Peptide Synthesizer (Perkin Elmer). Optionally, fragments of lipoxin A₄ receptor-like polypeptides can be separately synthesized and combined using chemical methods to produce a full-length molecule.

The newly synthesized peptide can be substantially purified by preparative high performance liquid chromatography (*e.g.*, Creighton, PROTEINS: STRUCTURES AND MOLECULAR PRINCIPLES, WH Freeman and Co., New York, N.Y., 1983). The composition of a synthetic lipoxin A₄ receptor-like polypeptide can be confirmed by amino acid analysis or sequencing *(e.g.,* the Edman degradation procedure; *see* Creighton, *supra*). Additionally, any portion of the amino acid sequence of the lipoxin A₄ receptor-like polypeptide can be altered during direct synthesis and/or combined using chemical methods with sequences from other proteins to produce a variant polypeptide or a fusion protein.

### Production of Altered Lipoxin A₄ Receptor-Like Polypeptides

As will be understood by those of skill in the art, it may be advantageous to produce lipoxin A₄ receptor-like polypeptide-encoding nucleotide sequences possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences disclosed herein can be engineered using methods generally known in the art to alter lipoxin A₄ receptor-like polypeptide-encoding sequences for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

### Antibodies

Any type of antibody known in the art can be generated to bind specifically to an epitope of a lipoxin A₄ receptor-like polypeptide. "Antibody" as used herein includes intact immunoglobulin molecules, as well as fragments thereof, such as Fab, F(ab')₂, and Fv, which are capable of binding an epitope of a lipoxin A₄ receptor-like polypeptide. Typically, at least 6, 8, 10, or 12 contiguous amino acids are required to form an epitope. However, epitopes which involve non-contiguous amino acids may require more, *e.g.*, at least 15, 25, or 50 amino acids.

An antibody which specifically binds to an epitope of a lipoxin A₄ receptor-like polypeptide can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the immunogen.

Typically, an antibody which specifically binds to a lipoxin A₄ receptor-like polypeptide provides a detection signal at least 5-, 10-, or 20-fold higher than a detection signal provided with other proteins when used in an immunochemical assay. Preferably, antibodies which specifically bind to lipoxin A₄ receptor-like polypeptides do not detect other proteins in immunochemical assays and can immunoprecipitate a lipoxin A₄ receptor- like polypeptide from solution.

Lipoxin A₄ receptor-like polypeptides can be used to immunize a mammal, such as a mouse, rat, rabbit, guinea pig, monkey, or human, to produce polyclonal antibodies. If desired, a lipoxin A₄ receptor-like polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (*e.g.*, aluminum hydroxide), and surface active substances (*e.g.* lysolecithin, pluronic polyols, polyanions, peptides, oil emulsions, keyhole limpet hemocyanin, and dinitrophenol). Among adjuvants used in humans, BCG (*bacilli Calmette-Guerin*) and *Corynebacterium parvum* are especially useful.

Monoclonal antibodies which specifically bind to a lipoxin A₄ receptor-like polypeptide can be prepared using any technique which provides for the production of antibody molecules by continuous cell lines in culture. These techniques include, but are not limited to, the hybridoma technique, the human B-cell hybridoma technique, and the EBV-hybridoma technique (Kohler *et al., Nature 256,* 495-497, 1985; Kozbor *et al., J. Immunol. Methods 81,* 31-42, 1985; Cote *et al., Proc. Natl. Acad Sci. 80,* 2026-2030, 1983; Cole *et al., Mol. Cell Biol. 62,* 109-120, 1984).

In addition, techniques developed for the production of "chimeric antibodies," the splicing of mouse antibody genes to human antibody genes to obtain a molecule with appropriate antigen specificity and biological activity, can be used (Morrison *et al., Proc. Natl. Acad Sci. 81,* 6851-6855, 1984; Neuberger *et al., Nature 312,* 604-608, 1984; Takeda *et al., Nature 314,* 452-454, 1985). Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Alternatively, humanized antibodies can be produced using recombinant methods, as described in GB2188638B. Antibodies which specifically bind to a lipoxin A₄ receptor-like polypeptide can contain antigen binding sites which are either partially or fully humanized, as disclosed in U.S. 5,565,332.

Alternatively, techniques described for the production of single chain antibodies can be adapted using methods known in the art to produce single chain antibodies which specifically bind to lipoxin A₄ receptor-like polypeptides. Antibodies with related specificity, but of distinct idiotypic composition, can be generated by chain shuffling from random combinatorial immunoglobin libraries (Burton, *Proc. Natl. Acad. Sci. 88,* 11120-23, 1991).

Single-chain antibodies also can be constructed using a DNA amplification method, such as PCR, using hybridoma cDNA as a template (Thirion *et al.,* 1996, *Eur. J. Cancer Prev. 5,* 507-11). Single-chain antibodies can be mono- or bispecific, and can be bivalent or tetravalent. Construction of tetravalent, bispecific single-chain antibodies is taught, for example, in Coloma & Morrison, 1997, *Nat. Biotechnol. 15,* 159-63. Construction of bivalent, bispecific single-chain antibodies is taught in Mallender & Voss, 1994, *J. Biol. Chem. 269,* 199-206.

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology (Verhaar *et al.,* 1995, *Int. J. Cancer 61,* 497-501; Nicholls *et al.,* 1993, *J. Immunol. Meth. 165,* 81-91).

Antibodies which specifically bind to lipoxin A₄ receptor-like polypeptides also can be produced by inducing *in vivo* production in the lymphocyte population or by screening immunoglobulin libraries or panels of highly specific binding reagents as disclosed in the literature (Orlandi *et al., Proc. Natl. Acad Sci. 86*, 3833-3837, 1989; Winter *et al., Nature 349,* 293-299, 1991).

Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which a lipoxin A₄ receptor-like polypeptide is bound. The bound antibodies can then be eluted from the column using a buffer with a high salt concentration.

### Antisense Oligonucleotides

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of lipoxin A₄ receptor-like protein gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters. *See* Brown, *Meth. Mol. Biol. 20,* 1-8, 1994; Sonveaux, *Meth. Mol. Biol. 26*, 1-72, 1994; Uhlmann *et al., Chem. Rev. 90*, 543-583, 1990.

Modifications of lipoxin A₄ receptor-like protein gene expression can be obtained by designing antisense oligonucleotides which will form duplexes to the control, 5', or regulatory regions of the lipoxin A₄ receptor-like protein gene. Oligonucleotides derived from the transcription initiation site, *e.g.*, between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature (*e.g., Gee et al.,* in Huber & Carr, MOLECULAR AND IMMUNOLOGIC APPROACHES, Futura Publishing Co., Mt. Kisco, N.Y., 1994). An antisense oligonucleotide also can be designed to block translation of mRNA by preventing the transcript from binding to ribosomes.

Precise complementarity is not required for successful complex formation between an antisense oligonucleotide and the complementary sequence of a lipoxin A₄ receptor-like polynucleotide. Antisense oligonucleotides which comprise, for example, 2, 3, 4, or 5 or more stretches of contiguous nucleotides which are precisely complementary to a lipoxin A₄ receptor-like polynucleotide, each separated by a stretch of contiguous nucleotides which are not complementary to adjacent lipoxin A₄ receptor-like protein nucleotides, can provide sufficient targeting specificity for lipoxin A₄ receptor-like protein mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular lipoxin A₄ receptor-like polynucleotide sequence.

Antisense oligonucleotides can be modified without affecting their ability to hybridize to a lipoxin A₄ receptor-like polynucleotide. These modifications can be internal or at one or both ends of the antisense molecule. For example, inter-nucleoside phosphate linkages can be modified by adding cholesteryl or diamine moieties with varying numbers of carbon residues between the amino groups and terminal ribose. Modified bases and/or sugars, such as arabinose instead of ribose, or a 3',5'-substituted oligonucleotide in which the 3' hydroxyl group or the 5' phosphate group are substituted, also can be employed in a modified antisense oligonucleotide. These modified oligonucleotides can be prepared by methods well known in the art.

*See, e.g.,* Agrawal *et al., Trends Biotechnol. 10,* 152-158, 1992; Uhlmann *et al., Chem. Rev. 90,* 543-584, 1990; Uhlmann *et al., Tetrahedron. Lett. 215,* 3539-3542, 1987.

### Ribozymes

Ribozymes are RNA molecules with catalytic activity. *See, e.g.,* Cech, *Science 236,* 1532-1539; 1987; Cech, *Ann. Rev. Biochem. 59*, 543-568; 1990, Cech, *Curr. Opin. Struct. Biol. 2,* 605-609; 1992, Couture & Stinchcomb, *Trends Genet. 12,* 510-515, 1996. Ribozymes can be used to inhibit gene function by cleaving an RNA sequence, as is known in the art (*e.g.*, Haseloff *et al.,* U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences.

The coding sequence of a lipoxin A₄ receptor-like polynucleotide can be used to generate ribozymes which will specifically bind to mRNA transcribed from the lipoxin A₄ receptor-like polynucleotide. Methods of designing and constructing ribozymes which can cleave other RNA molecules in trans in a highly sequence specific manner have been developed and described in the art (*see* Haseloff *et al. Nature 334,* 585-591, 1988). For example, the cleavage activity of ribozymes can be targeted to specific RNAs by engineering a discrete "hybridization" region into the ribozyme. The hybridization region contains a sequence complementary to the target RNA and thus specifically hybridizes with the target (see, for example, Gerlach *et al.,* EP 321,201).

Specific ribozyme cleavage sites within a lipoxin A₄ receptor-like protein RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate lipoxin A₄ receptor-like protein RNA targets also can be evaluated by testing accessibility to hybridization with complementary oligonucleotides using ribonuclease protection assays. The nucleotide sequences shown in SEQ ID NO. 1 and their complements provide sources of suitable hybridization region sequences. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease lipoxin A₄ receptor-like protein expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff *et al.,* U.S. Patent 5,641,673, ribozymes can be engineered so that ribozyme expression will occur in response to factors which induce expression of a target gene. Ribozymes also can be engineered to provide an additional level of regulation, so that destruction of mRNA occurs only when both a ribozyme and a target gene are induced in the cells.

### Screening Methods

Disclosed are assays for screening test compounds which bind to or modulate the activity of a lipoxin A₄ receptor-like polypeptide or a lipoxin A₄ receptor-like polynucleotide. A test compound preferably binds to a lipoxin A₄ receptor-like polypeptide or polynucleotide. More preferably, a test compound decreases or increases a lipoxin A₄ receptor-like protein activity of a lipoxin A₄ receptor-like polypeptide or expression of a lipoxin A₄ receptor-like polynucleotide by at least about 10, preferably about 50, more preferably about 75, 90, or 100% relative to the absence of the test compound.

### Test Compounds

Test compounds can be pharmacologic agents already known in the art or can be compounds previously unknown to have any pharmacological activity. The compounds can be naturally occurring or designed in the laboratory. They can be isolated from microorganisms, animals, or plants, and can be produced recombinantly, or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. *See* Lam, *Anticancer Drug Des. 12,* 145, 1997.

Methods for the synthesis of molecular libraries are well known in the art (*see,* for example, DeWitt *el al., Proc. Natl. Acad. Sci. U.S.A. 90,* 6909, 1993; Erb *et al. Proc. Natl. Acad Sci. U.S.A. 91,* 11422, 1994; Zuckermann *et al., J. Med. Chem. 37,* 2678, 1994; Cho *et al., Science 261,* 1303, 1993; Carell *et al., Angew. Chem. Int. Ed. Engl. 33*, 2059, 1994; Carell *et al., Angew. Chem. Int. Ed. Engl. 33,* 2061; Gallop *et al., J. Med. Chem. 37,* 1233, 1994). Libraries of compounds can be presented in solution (*see, e.g.,* Houghten, *Biotechniques 13,* 412-421, 1992), or on beads (Lam, *Nature 354,* 82-84, 1991), chips (Fodor, *Nature 364,* 555-556, 1993), bacteria or spores (Ladner, U.S. Patent 5,223,409), plasmids (Cull *et al., Proc. Natl. Acad Sci. U.S.A. 89*, 1865-1869, 1992), or phage (Scott & Smith, *Science 249,* 386-390, 1990; Devlin, *Science 249*, 404-406, 1990); Cwirla *et al., Proc. Natl. Acad Sci. 97,* 6378-6382, 1990; Felici, *J. Mol. Biol. 222*, 301-310, 1991; and Ladner, U.S. Patent 5,223,409).

### High Throughput Screening

Test compounds can be screened for the ability to bind to lipoxin A₄ receptor-like polypeptides or polynucleotides or to affect lipoxin A₄ receptor-like protein activity or lipoxin A₄ receptor-like protein gene expression using high throughput screening. Using high throughput screening, many discrete compounds can be tested in parallel so that large numbers of test compounds can be quickly screened. The most widely established techniques utilize 96-well microtiter plates. The wells of the microtiter plates typically require assay volumes that range from 50 to 500 1. In addition to the plates, many instruments, materials, pipettors, robotics, plate washers, and plate readers are commercially available to fit the 96-well format.

Alternatively, "free format assays," or assays that have no physical barrier between samples, can be used. For example, an assay using pigment cells (melanocytes) in a simple homogeneous assay for combinatorial peptide libraries is described by Jayawickreme *et al., Proc. Natl. Acad Sci. U.S.A. 19,* 1614-18 (1994). The cells are placed under agarose in petri dishes, then beads that carry combinatorial compounds are placed on the surface of the agarose. The combinatorial compounds are partially released the compounds from the beads. Active compounds can be visualized as dark pigment areas because, as the compounds diffuse locally into the gel matrix, the active compounds cause the cells to change colors.

Another example of a free format assay is described by Chelsky, "Strategies for Screening Combinatorial Libraries: Novel and Traditional Approaches," reported at the First Annual Conference of The Society for Biomolecular Screening in Philadephia, Pa. (Nov. 7-10, 1995). Chelsky placed a simple homogenous enzyme assay for carbonic anhydrase inside an agarose gel such that the enzyme in the gel would cause a color change throughout the gel. Thereafter, beads carrying combinatorial compounds via a photolinker were placed inside the gel and the compounds were partially released by UV-light. Compounds that inhibited the enzyme were observed as local zones of inhibition having less color change.

Yet another example is described by Salmon *et al., Molecular Diversity 2,* 57-63 (1996). In this example, combinatorial libraries were screened for compounds that had cytotoxic effects on cancer cells growing in agar.

Another high throughput screening method is described in Beutel *et al.,* U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

### Binding Assays

For binding assays, the test compound is preferably a small molecule which binds to and occupies the active site of the lipoxin A₄ receptor-like polypeptide, thereby making the ligand binding site inaccessible to substrate such that normal biological activity is prevented. Examples of such small molecules include, but are not limited to, small peptides or peptide-like molecules. Potential ligands which bind to a polypeptide of the invention include, but are not limited to, the natural ligands of known lipoxin A₄ receptor- like proteins and analogues or derivatives thereof.

In binding assays, either the test compound or the lipoxin A₄ receptor-like polypeptide can comprise a detectable label, such as a fluorescent, radioisotopic, chemiluminescent, or enzymatic label, such as horseradish peroxidase, alkaline phosphatase, or luciferase. Detection of a test compound which is bound to the lipoxin A₄ receptor-like polypeptide can then be accomplished, for example, by direct counting of radioemmission, by scintillation counting, or by determining conversion of an appropriate substrate to a detectable product.

Alternatively, binding of a test compound to a lipoxin A₄ receptor-like polypeptide can be determined without labeling either of the interactants. For example, a microphysiometer can be used to detect binding of a test compound with a lipoxin A₄ receptor-like polypeptide. A microphysiometer (*e.g.,* Cytosensor^{™}) is an analytical instrument that measures the rate at which a cell acidifies its environment using a light-addressable potentiometric sensor (LAPS). Changes in this acidification rate can be used as an indicator of the interaction between a test compound and a lipoxin A₄ receptor- like polypeptide (McConnell *et al., Science 257,* 1906-1912, 1992).

Determining the ability of a test compound to bind to a lipoxin A₄ receptor-like polypeptide also can be accomplished using a technology such as real-time Bimolecular Interaction Analysis (BIA) (Sjolander & Urbaniczky, *Anal. Chem. 63,* 2338-2345, 1991, and Szabo *et al., Curr. Opin. Struct. Biol. 5,* 699-705, 1995). BIA is a technology for studying biospecific interactions in real time, without labeling any of the interactants (*e.g.,* BIAcore^{™}). Changes in the optical phenomenon surface plasmon resonance (SPR) can be used as an indication of real-time reactions between biological molecules.

In yet another aspect of the invention, a lipoxin A₄ receptor-like polypeptide can be used as a "bait protein" in a two-hybrid assay or three-hybrid assay (*see, e.g.*, U.S. Patent 5,283,317; Zervos *et al., Cell 72,* 223-232, 1993; Madura *et al., J. Biol. Chem. 268,* 12046-12454, 1993; Bartel *et al., Biotechniques 14,* 920-924, 1993; Iwabuchi *et al., Oncogene 8,* 1693-1696, 1993; and Brent W094/10300), to identify other proteins which bind to or interact with the lipoxin A₄ receptor-like polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a lipoxin A₄ receptor-like polypeptide can be fused to a polynucleotide encoding the DNA binding domain of a known transcription factor (*e.g.*, GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (*e.g*., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the lipoxin A₄ receptor-like polypeptide.

It may be desirable to immobilize either the lipoxin A₄ receptor-like polypeptide (or polynucleotide) or the test compound to facilitate separation of bound from unbound forms of one or both of the interactants, as well as to accommodate automation of the assay. Thus, either the lipoxin A₄ receptor-like polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach the lipoxin A₄ receptor-like polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a lipoxin A₄ receptor-like polypeptide (or polynucleotide) can be accomplished in any vessel suitable for containing the reactants. Examples of such vessels include microtiter plates, test tubes, and microcentrifuge tubes.

In one embodiment, the lipoxin A₄ receptor-like polypeptide is a fusion protein comprising a domain that allows the lipoxin A₄ receptor-like polypeptide to be bound to a solid support. For example, glutathione-S-transferase fusion proteins can be adsorbed onto glutathione sepharose beads (Sigma Chemical, St. Louis, Mo.) or glutathione derivatized microtiter plates, which are then combined with the test compound or the test compound and the non-adsorbed lipoxin A₄ receptor-like polypeptide; the mixture is then incubated under conditions conducive to complex formation (*e.g*., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either a lipoxin A₄ receptor-like polypeptide (or polynucleotide) or a test compound can be immobilized utilizing conjugation of biotin and streptavidin. Biotinylated lipoxin A₄ receptor-like polypeptides (or polynucleotides) or test compounds can be prepared from biotin-NHS(N-hydroxysuccinimide) using techniques well known in the art (*e.g.*, biotinylation kit, Pierce Chemicals, Rockford, III.) and immobilized in the wells of streptavidin-coated 96 well plates (Pierce Chemical). Alternatively, antibodies which specifically bind to a lipoxin A₄ receptor-like polypeptide, polynucleotide, or a test compound, but which do not interfere with a desired binding site, such as the active site of the lipoxin A₄ receptor-like polypeptide, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to the lipoxin A₄ receptor-like polypeptide or test compound, enzyme-linked assays which rely on detecting an activity of the lipoxin A₄ receptor-like polypeptide, and SDS gel electrophoresis under non-reducing conditions.

Screening for test compounds which bind to a lipoxin A₄ receptor-like polypeptide or polynucleotide also can be carried out in an intact cell. Any cell which comprises a lipoxin A₄ receptor-like polypeptide or polynucleotide can be used in a cell-based assay system. A lipoxin A₄ receptor-like polynucleotide can be naturally occurring in the cell or can be introduced using techniques such as those described above. Binding of the test compound to a lipoxin A₄ receptor-like polypeptide or polynucleotide is determined as described above.

### Lipoxin A₄ Receptor-Like Protein Assays

Test compounds can be tested for the ability to increase or decrease signal transduction mediated by a lipoxin A₄ receptor-like polypeptide. Functional assays include the use of cells which express the G-protein coupled receptor (for example, transfected CHO cells) in a system which measures extracellular pH changes caused by receptor activation (*see, e.g., Science 246,* 181-296, 1989). For example, compounds may be contacted with a cell which expresses the receptor polypeptide of the present invention and a second messenger response, *e.g.*, signal transduction or pH changes, can be measured to determine whether the potential compound activates or inhibits the receptor. Functional assays can be conducted after contacting either a purified lipoxin A₄ receptor-like polypeptide, a cell membrane preparation, or an intact cell with a test compound. A test compound which decreases a lipoxin A₄ receptor-like protein activity by at least about 10, preferably about 50, more preferably about 75, 90, or 100% is identified as a potential agent for decreasing lipoxin A₄ receptor-like protein activity. A test compound which increases lipoxin A₄ receptor-like protein activity by at least about 10, preferably about 50, more preferably about 75, 90, or 100% is identified as a potential agent for increasing lipoxin A₄ receptor-like protein activity.

Another screening procedure involves the use of melanophores which are transfected to express the G-protein coupled receptor of the present invention. Such a screening technique is described in WO 92/01810. Thus, for example, such an assay may be employed for screening for a compound which inhibits activation of the receptor polypeptide of the present invention by contacting the melanophore cells which encode the receptor with both the receptor ligand and a compound to be screened. Inhibition of the signal generated by the ligand indicates that a compound is a potential antagonist for the receptor, *i.e.,* inhibits activation of the receptor. The screen may be employed for identifying a compound which activates the receptor by contacting such cells with compounds to be screened and determining whether each compound generates a signal, *i.e.,* activates the receptor.

Yet another such screening technique involves introducing RNA encoding a G-protein coupled receptor into *Xenopus* oocytes to express the receptor transiently. The receptor oocytes can then be contacted with the receptor ligand and a compound to be screened, followed by detection of inhibition or activation of a calcium signal in the case of screening for compounds which are thought to inhibit activation of the receptor.

Another screening technique involves expressing the G-protein coupled receptor in cells in which the receptor is linked to a phospholipase C or D. Such cells include endothelial cells, smooth muscle cells, embryonic kidney cells, etc. The screening may be accomplished as described above by quantifying the degree of activation of the receptor from changes in the phospholipase activity.

Detailed examples of functional assays such as those described above are provided in the specific examples below.

### Lipoxin A₄ Receptor-Like Gene Expression

In another embodiment, test compounds which increase or decrease lipoxin A₄ receptor-like protein gene expression are identified. A lipoxin A₄ receptor-like polynucleotide is contacted with a test compound, and the expression of an RNA or polypeptide product of the lipoxin A₄ receptor-like polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of lipoxin A₄ receptor-like protein mRNA or polypeptide expression in the cells can be determined by methods well known in the art for detecting mRNA or polypeptide. Either qualitative or quantitative methods can be used. The presence of polypeptide products of a lipoxin A₄ receptor-like polynucleotide can be determined, for example, using a variety of techniques known in the art, including immunochemical methods such as radioimmunoassay, Western blotting, and immuno-histochemistry. Alternatively, polypeptide synthesis can be determined *in vivo,* in a cell culture, or in an *in vitro* translation system by detecting incorporation of labeled amino acids into a lipoxin A₄ receptor-like polypeptide.

Such screening can be carried out either in a cell-free assay system or in an intact cell. Any cell which expresses a lipoxin A₄ receptor-like polynucleotide can be used in a cell-based assay system. The lipoxin A₄ receptor-like polynucleotide can be naturally occurring in the cell or can be introduced using techniques such as those described above. Either a primary culture or an established cell line, such as CHO or human embryonic kidney 293 cells, can be used.

### Diagnostic and Therapeutic Indications and Methods

GPCRs are ubiquitous in mammals, including humans, and are responsible for many biological functions, including many pathologies. Accordingly, it is desirable to find compounds and drugs which stimulate GPCRs on the one hand and which can inhibit a GPCRs on the other hand. For example, compounds which activate a GPCR may be employed for therapeutic purposes, such as the treatment of asthma, Parkinson's disease, acute heart failure, urinary retention, and osteoporosis. In particular, compounds which activate the receptors of the present invention are useful in treating various cardiovascular ailments such as caused by the lack of pulmonary blood flow or hypertension. In addition these compounds may also be used in treating various physiological disorders relating to abnormal control of fluid and electrolyte homeostasis and in diseases associated with abnormal angiotensin-induced aldosterone secretion.

In general, compounds which inhibit activation of a GPCR may be employed for a variety of therapeutic purposes, for example, for the treatment of hypotension and/or hypertension, angina pectoris, myocardial infarction, ulcers, asthma, allergies, benign prostatic hypertrophy, and psychotic and neurological disorders including schizophrenia, manic excitement, depression, delirium, dementia or severe mental retardation, dyskinesias, such as Huntington's disease or Tourett's syndrome, among others. Compounds which inhibit GPCRs have also been useful in reversing endogenous anorexia and in the control of bulimia. In particular, compounds which inhibit the activation of the receptors of the present invention are useful in treating various cardiovascular ailments such as caused by excessive pulmonary blood flow or hypotension. In addition these compounds may also be used in treating various physiological disorders relating to abnormal control of fluid and electrolyte homeostasis and in diseases associated with abnormal angiotensin-induced aldosterone secretion.

Modulation of lipoxin A₄ receptor-like protein binding to its naturally occurring ligand can be used, for example, in the control of hemostasis, vascular reactivity, especially vasoconstriction, and anaphylactic and allergic reactions in mammals, preferably in humans (*see* U.S. Patent 5,079,261). For example, blocking the binding of lipoxin A₄ receptor-like protein's ligand to the receptor can be used to reduce the inflammatory response of neutrophils. Alternatively, agonists of human lipoxin A₄ receptor-like protein can be used to induce or enhance this inflammatory response.

Novel agents identified by the screening assays described above can be used to regulate human lipoxin A₄ receptor-like protein activity. Accordingly, it is disclosed to use a test compound identified as described herein in an appropriate animal model. For example, an agent identified as described herein (*e.g.,* a modulating agent, an antisense nucleic acid molecule, a specific antibody, ribozyme, or a protein-binding partner) can be used in an animal model to determine the efficacy, toxicity, or side effects of treatment with such an agent. Alternatively, an agent identified as described herein can be used in an animal model to determine the mechanism of action of such an agent.

A reagent which affects lipoxin A₄ receptor-like protein activity can be administered to a human cell, either *in vitro* or *in vivo,* to reduce lipoxin A₄ receptor-like protein activity. The reagent preferably binds to an expression product of a human lipoxin A₄ receptor-like protein gene. If the expression product is a protein, the reagent is preferably an antibody. For treatment of human cells *ex vivo,* an antibody can be added to a preparation of stem cells which have been removed from the body. The cells can then be replaced in the same or another human body, with or without clonal propagation, as is known in the art.

The reagent can be delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.

A liposome useful comprises a lipid composition that is capable of fusing with the plasma membrane of the targeted cell to deliver its contents to the cell. Preferably, the transfection efficiency of a liposome is about 0.5 µg of DNA per 16 nmole of liposome delivered to about 10⁶ cells, more preferably about 1.0 µg of DNA per 16 nmol of liposome delivered to about 10⁶ cells, and even more preferably about 2.0 µg of DNA per 16 nmol of liposome delivered to about 10⁶ cells. Preferably, a liposome is between about 100 and 500 nm, more preferably between about 150 and 450 nm, and even more preferably between about 200 and 400 nm in diameter.

Suitable liposomes include those liposomes standardly used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a tumor cell, such as a tumor cell ligand exposed on the outer surface of the liposome.

Complexing a liposome with a reagent such as an antisense oligonucleotide or ribozyme can be achieved using methods which are standard in the art (see, for example, U.S. Patent 5,705,151). Preferably, from about 0.1 µg to about 10 µg of polynucleotide is combined with about 8 nmol of liposomes, more preferably from about 0.5 µg to about 5 µg of polynucleotides are combined with about 8 nmol liposomes, and even more preferably about 1.0 µg of polynucleotides is combined with about 8 nmol liposomes.

Antibodies can be delivered to specific tissues *in vivo* using receptor-mediated targeted delivery. Receptor-mediated DNA delivery techniques are taught in, for example, Findeis *et al. Trends in Biotechnol. 11,* 202-05 (1993); Chiou *et al.,* GENE THERAPEUTICS: METHODS AND APPLICATIONS OF DIRECT GENE TRANSFER (J.A. Wolff, ed.) (1994); Wu & Wu, *J. Biol. Chem. 263,* 621-24 (1988); Wu *et al., J. Biol. Chem. 269,* 542-46 (1994); Zenke *et al., Proc. Natl. Acad. Sci. U.S.A. 87,* 3655-59 (1990); Wu *et al., J. Biol. Chem. 266,* 338-42 (1991).

### Determination of a Therapeutically Effective Dose

The determination of a therapeutically effective dose is well within the capability of those skilled in the art. A therapeutically effective dose refers to that amount of active ingredient which increases or decreases lipoxin A₄ receptor-like protein activity relative to the lipoxin A₄ receptor-like protein activity which occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, *e.g.*, ED₅₀ (the dose therapeutically effective in 50% of the population) and LD₅₀ (the dose lethal to 50% of the population), can be determined by standard pharmaceutical procedures in cell cultures or experimental animals. The dose ratio of toxic to therapeutic effects is the therapeutic index, and it can be expressed as the ratio, LD₅₀/ED₅₀.

Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the ED₅₀ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun," and DEAE- or calcium phosphate-mediated transfection.

Effective *in vivo* dosages of an antibody are in the range of about 5 µg to about 50 µg/kg, about 50 µg to about 5 mg/kg, about 100 µg to about 500 µg/kg of patient body weight, and about 200 to about 250 µg/kg of patient body weight. For administration of polynucleotides encoding single-chain antibodies, effective *in vivo* dosages are in the range of about 100 ng to about 200 ng, 500 ng to about 50 mg, about 1 µg to about 2 mg, about 5 µg to about 500 µg, and about 20 µg to about 100 µg of DNA.

If the expression product is mRNA, the reagent is preferably an antisense oligonucleotide or a ribozyme. Polynucleotides which express antisense oligonucleotides or ribozymes can be introduced into cells by a variety of methods, as described above.

Preferably, a reagent reduces expression of a lipoxin A₄ receptor-like protein gene or the activity of a lipoxin A₄ receptor-like polypeptide by at least about 10, preferably about 50, more preferably about 75, 90, or 100% relative to the absence of the reagent. The effectiveness of the mechanism chosen to decrease the level of expression of a lipoxin A₄ receptor-like protein gene or the activity of a lipoxin A₄ receptor-like polypeptide can be assessed using methods well known in the art, such as hybridization of nucleotide probes to lipoxin A₄ receptor-like protein-specific mRNA, quantitative RT-PCR, immunologic detection of a lipoxin A₄ receptor-like polypeptide, or measurement of lipoxin A₄ receptor- like protein activity.

Any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

Any of the therapeutic methods described above can be applied to any subject in need of such therapy, including, for example, mammals such as dogs, cats, cows, horses, rabbits, monkeys, and most preferably, humans.

### EXAMPLE 1

### Detection of lipoxin A₄ receptor-like activity

The polynucleotide of SEQ ID NO. 1 is inserted into the expression vector pCEV4 and the expression vector pCEV4-lipoxin A₄ receptor-like polypeptide obtained is transfected into human embryonic kidney 293 cells. The cells are scraped from a culture flask into 5 ml of Tris HCl, 5 mM EDTA, pH 7.5, and lysed by sonication. Cell lysates are centrifuged at 1000 rpm for 5 minutes at 4°C. The supernatant is centrifuged at 30,000 x g for 20 minutes at 4°C. The pellet is suspended in binding buffer containing 50 mM Tris HCl, 5 mM MgSO₄, 1 mM EDTA, 100 mM NaCl, pH 7.5, supplemented with 0.1 % BSA, 2 µg/ml aprotinin, 0.5 mg/ml leupeptin, and 10 µg/ml phosphoramidon. Optimal membrane suspension dilutions, defined as the protein concentration required to bind less than 10 % of an added radioligand, i.e. ¹²⁵I-labeled lipoxin A₄, are added to 96-well polypropylene microtiter plates containing ligand, non-labeled peptides, and binding buffer to a final volume of 250 µl..

In equilibrium saturation binding assays, membrane preparations are incubated in the presence of increasing concentrations (0.1 nM to 4 nM) of ¹²⁵I ligand.

Binding reaction mixtures are incubated for one hour at 30°C. The reaction is stopped by filtration through GF/B filters treated with 0-5% polyethyleneimine, using a cell harvester. Radioactivity is measured by scintillation counting, and data are analyzed by a computerized non-linear regression program. Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. The lipoxin A₄ receptor-like activity of the polypeptide comprising the amino acid sequence of SEQ ID NO. 2 is demonstrated.

### EXAMPLE 2

### Radioligand binding assays

Human embryonic kidney 293 cells transfected with a polynucleotide which expresses human lipoxin A₄ receptor-like protein are scraped from a culture flask into 5 ml of Tris HCl, 5 mM EDTA, pH 7.5, and lysed by sonication. Cell lysates are centrifuged at 1000 rpm for 5 minutes at 4°C. The supernatant is centrifuged at 30,000 x g for 20 minutes at 4°C. The pellet is suspended in binding buffer containing 50 mM Tris HCl, 5 mM MgSO₄, 1 mM EDTA, 100 mM NaCl, pH 7.5, supplemented with 0.1 % BSA, 2 µg/ml aprotinin, 0.5 mg/ml leupeptin, and 10 g/ml phosphoramidon. Optimal membrane suspension dilutions, defined as the protein concentration required to bind less than 10 % of the added radioligand, i.e. ¹²⁵I-labeled lipoxin A₄, are added to 96-well polypropylene microtiter plates containing ligand or test compound, non-labeled peptides, and binding buffer to a final volume of 250 µl.

In equilibrium saturation binding assays, membrane preparations are incubated in the presence of increasing concentrations (0.1 nM to 4 nM) of ¹²⁵I ligand or test compound (specific activity 2200 Ci/mmol). The binding affinities of different test compounds are determined in equilibrium competition binding assays, using 0.1 nM ¹²⁵I-peptide in the presence of twelve different concentrations of each test compound.

Binding reaction mixtures are incubated for one hour at 30°C. The reaction is stopped by filtration through GF/B filters treated with 0.5% polyethyleneimine, using a cell harvester. Radioactivity is measured by scintillation counting, and data are analyzed by a computerized non-linear regression program.

Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. A test compound which increases the radioactivity of membrane protein by at least 15% relative to radioactivity of membrane protein which was not incubated with a test compound is identified as a compound which binds to a human lipoxin A₄ receptor-like polypeptide.

### EXAMPLE 3

### Effect of a test compound on human lipoxin A₄ receptor-like protein-mediated cyclic AMP formation

Receptor-mediated inhibition of cAMP formation can be assayed in LM(tk-) cells which express human lipoxin A₄ receptor-like protein. Cells are plated in 96-well plates and incubated in Dulbecco's phosphate buffered saline (PBS) supplemented with 10 mM HEPES, 5 mM theophylline, 2 µg/ml aprotinin, 0.5 mg/ml leupeptin, and 10 g/ml phosphoramidon for 20 minutes at 37 °C in 5% CO2. A test compound is added and incubated for an additional 10 minutes at 37°C. The medium is aspirated, and the reaction is stopped by the addition of 100 mM HCl. The plates are stored at 4°C for 15 minutes. cAMP content in the stopping solution is measured by radioimmunoassay.

Radioactivity is quantified using a gamma counter equipped with data reduction software. A test compound which decreases radioactivity of the contents of a well relative to radioactivity of the contents of a well in the absence of the test compound is identified as a potential inhibitor of cAMP formation. A test compound which increases radioactivity of the contents of a well relative to radioactivity of the contents of a well in the absence of the test compound is identified as a potential enhancer of cAMP formation.

### EXAMPLE 4

### Effect of a test compound on the mobilization of intracellular calcium

Intracellular free calcium concentration can be measured by microspectrofluorometry using the fluorescent indicator dye Fura-2/AM (Bush *et al., J. Neurochem. 57,* 562-74, 1991). Stably transfected cells are seeded onto a 35 mm culture dish containing a glass coverslip insert. Cells are washed with HBS , incubated with a test compound, and loaded with 100 µl of Fura-2/AM (10 µM) for 20-40 minutes. After washing with HBS to remove the Fura-2/AM solution, cells are equilibrated in HBS for 10-20 minutes. Cells are then visualized under the 40X objective of a Leitz Fluovert FS microscope.

Fluorescence emission is determined at 510 nM, with excitation wavelengths alternating between 340 nM and 380 nM. Raw fluorescence data are converted to calcium concentrations using standard calcium concentration curves and software analysis techniques. A test compound which increases the fluorescence by at least 15% relative to fluorescence in the absence of a test compound is identified as a compound which mobilizes intracellular calcium.

### EXAMPLE 5

### Effect of a test compound on phosphoinositide metabolism

LM(tk-) cells which stably express human lipoxin A₄ receptor-like protein cDNA are plated in 96-well plates and grown to confluence. The day before the assay, the growth medium is changed to 100 µl of medium containing 1% serum and 0.5 µCi ³H-myinositol. The plates are incubated overnight in a CO₂ incubator (5% CO₂ at 37 °C). Immediately before the assay, the medium is removed and replaced by 200 µl of PBS containing 10 mM LiCl, and the cells are equilibrated with the new medium for 20 minutes. During this interval, cells also are equilibrated with antagonist, added as a 10 µl aliquot of a 20-fold concentrated solution in PBS.

The ³H-inositol phosphate accumulation from inositol phospholipid metabolism is started by adding 10 µl of a solution containing a test compound. To the first well 10 µl are added to measure basal accumulation. Eleven different concentrations of test compound are assayed in the following 11 wells of each plate row. All assays are performed in duplicate by repeating the same additions in two consecutive plate rows.

The plates are incubated in a CO₂ incubator for one hour. The reaction is terminated by adding 15 µl of 50% v/v trichloroacetic acid (TCA), followed by a 40 minute incubation at 4°C. After neutralizing TCA with 40 µl of 1 M Tris, the content of the wells is transferred to a Multiscreen HV filter plate (Millipore) containing Dowex AG1-X8 (200-400 mesh, formate form). The filter plates are prepared by adding 200 µl of Dowex AG1-X8 suspension (50% v/v, water:resin) to each well. The filter plates are placed on a vacuum manifold to wash or elute the resin bed. Each well is washed 2 times with 200 µl of water, followed by 2 x 200 µl of 5 mM sodium tetraborate/60 mM ammonium formate.

The ³H-IPs are eluted into empty 96-well plates with 200 µl of 1.2 M ammonium formate/0.1 formic acid. The content of the wells is added to 3 ml of scintillation cocktail, and radioactivity is determined by liquid scintillation counting.

### EXAMPLE 6

### Receptor Binding Methods

Standard Binding Assays. Binding assays are carried out in a binding buffer containing 50 mM HEPES, pH 7.4, 0.5% BSA, and 5 mM MgCl₂. The standard assay for radioligand (*e.g.,* ¹²⁵I-lipoxin A₄, -¹²⁵I-lipoxin A₄, analog, or -test compound) binding to membrane fragments comprising lipoxin A₄ receptor-like polypeptides is carried out as follows in 96 well microtiter plates (*e.g.,* Dynatech Immulon II Removawell plates). Radioligand is diluted in binding buffer+ PMSF/Baci to the desired cpm per 50 µl, then 50 µl aliquots are added to the wells. For non-specific binding samples, 5 µl of 40 µM cold ligand also is added per well. Binding is initiated by adding 150 µl per well of membrane diluted to the desired concentration (10-30 µg membrane protein/well) in binding buffer+ PMSF/Baci. Plates are then covered with Linbro mylar plate sealers (Flow Labs) and placed on a Dynatech Microshaker II. Binding is allowed to proceed at room temperature for 1-2 hours and is stopped by centrifuging the plate for 15 minutes at 2,000 x g. The supernatants are decanted, and the membrane pellets are washed once by addition of 200 µl of ice cold binding buffer, brief shaking, and recentrifugation. The individual wells are placed in 12 x 75 mm tubes and counted in an LKB Gammamaster counter (78% efficiency). Specific binding by this method is identical to that measured when free ligand is removed by rapid (3-5 seconds) filtration and washing on polyethyleneimine-coated glass fiber filters.

Three variations of the standard binding assay are also used.
1. Competitive radioligand binding assays with a concentration range of cold ligand vs. ¹²⁵I-labeled ligand are carried out as described above with one modification. All dilutions of ligands being assayed are made in 40X PMSF/Baci to a concentration 40X the final concentration in the assay. Samples of peptide (5 µl each) are then added per microtiter well. Membranes and radioligand are diluted in binding buffer without protease inhibitors. Radioligand is added and mixed with cold ligand, and then binding is initiated by addition of membranes.
2. Chemical cross-linking of radioligand with receptor is done after a binding step identical to the standard assay. However, the wash step is done with binding buffer minus BSA to reduce the possibility of non-specific cross-linking of radioligand with BSA. The cross-linking step is carried out as described below.
3. Larger scale binding assays to obtain membrane pellets for studies on solubilization of receptor:ligand complex and for receptor purification are also carried out. These are identical to the standard assays except that (a) binding is carried out in polypropylene tubes in volumes from 1-250 ml, (b) concentration of membrane protein is always 0.5 mg/ml, and (c) for receptor purification, BSA concentration in the binding buffer is reduced to 0.25%, and the wash step is done with binding buffer without BSA, which reduces BSA contamination of the purified receptor.

### EXAMPLE 7

### Chemical Cross-Linking of Radioligand to Receptor

After a radioligand binding step as described above, membrane pellets are resuspended in 200 µl per microtiter plate well of ice-cold binding buffer without BSA. Then 5 µl per well of 4 mM N-5-azido-2-nitrobenzoyloxysuccinimide (ANB-NOS, Pierce) in DMSO is added and mixed. The samples are held on ice and UV-irradiated for 10 minutes with a Mineralight R-52G lamp (UVP Inc., San Gabriel, Calif.) at a distance of 5-10 cm. Then the samples are transferred to Eppendorf microfuge tubes, the membranes pelleted by centrifugation, supernatants removed, and membranes solubilized in Laemmli SDS sample buffer for polyacrylamide gel electrophoresis (PAGE). PAGE is carried out as described below. Radiolabeled proteins are visualized by autoradiography of the dried gels with Kodak XAR film and Dupont image intensifier screens.

### EXAMPLE 8

### Membrane Solubilization

Membrane solubilization is carried out in buffer containing 25 mM Tris, pH 8, 10% glycerol (w/v) and 0.2 mM CaCl₂ (solubilization buffer). The highly soluble detergents including Triton X-100, deoxycholate, deoxycholate:lysolecithin, CHAPS, and zwittergent are made up in solubilization buffer at 10% concentrations and stored as frozen aliquots. Lysolecithin is made up fresh because of insolubility upon freeze-thawing and digitonin is made fresh at lower concentrations due to its more limited solubility.

To solubilize membranes, washed pellets after the binding step are resuspended free of visible particles by pipetting and vortexing in solubilization buffer at 100,000 x g for 30 minutes. The supernatants are removed and held on ice and the pellets are discarded.

### EXAMPLE 9

### Assay of Solubilized Receptors

After binding of ¹²⁵I ligands and solubilization of the membranes with detergent, the intact R:L complex can be assayed by four different methods. All are carried out on ice or in a cold room at 4-10°C.).
1. Column chromatography (Knuhtsen *et al., Biochem. J. 254,* 641-647, 1988). Sephadex G-50 columns (8 x 250 mm) are equilibrated with solubilization buffer containing detergent at the concentration used to solubilize membranes and 1 mg/ml bovine serum albumin. Samples of solubilized membranes (0.2-0.5 ml) are applied to the columns and eluted at a flow rate of about 0.7 ml/minute. Samples (0.18 ml) are collected. Radioactivity is determined in a gamma counter. Void volumes of the columns are determined by the elution volume of blue dextran. Radioactivity eluting in the void volume is considered bound to protein. Radioactivity eluting later, at the same volume as free ¹²⁵I ligands, is considered non-bound.
2. Polyethyleneglycol precipitation (Cuatrecasas, *Proc. Natl. Acad Sci. USA 69,* 318-322, 1972). For a 100 1 sample of solubilized membranes in a 12 x 75 mm polypropylene tube, 0.5 ml of 1% (w/v) bovine gamma globulin (Sigma) in 0.1 M sodium phosphate buffer is added, followed by 0.5 ml of 25% (w/v) polyethyleneglycol (Sigma) and mixing. The mixture is held on ice for 15 minutes. Then 3 ml of 0.1 M sodium phosphate, pH 7.4, is added per sample. The samples are rapidly (1-3 seconds) filtered over Whatman GF/B glass fiber filters and washed with 4 ml of the phosphate buffer. PEG-precipitated receptor : ¹²⁵I-ligand complex is determined by gamma counting of the filters.
3. GFB/PEI filter binding (Bruns *et al., Analytical Biochem. 132,* 74-81, 1983). Whatman GF/B glass fiber filters are soaked in 0.3% polyethyleneimine (PEI, Sigma) for 3 hours. Samples of solubilized membranes (25-100 1) are replaced in 12 x 75 mm polypropylene tubes. Then 4 ml of solubilization buffer without detergent is added per sample and the samples are immediately filtered through the GFB/PEI filters (1-3 seconds) and washed with 4 ml of solubilization buffer. CPM of receptor : ¹²⁵I-ligand complex adsorbed to filters are determined by gamma counting.
4. Charcoal/Dextran (Paul and Said, *Peptides 7[Suppl. 1]*,147-149, 1986). Dextran T70 (0.5 g, Pharmacia) is dissolved in 1 liter of water, then 5 g of activated charcoal (Norit A, alkaline; Fisher Scientific) is added. The suspension is stirred for 10 minutes at room temperature and then stored at 4°C. until use. To measure R:L complex, 4 parts by volume of charcoal/dextran suspension are added to 1 part by volume of solubilized membrane.
   The samples are mixed and held on ice for 2 minutes and then centrifuged for 2 minutes at 11,000 x g in a Beckman microfuge. Free radioligand is adsorbed charcoal/dextran and is discarded with the pellet. Receptor : ¹²⁵I-ligand complexes remain in the supernatant and are determined by gamma counting.

### EXAMPLE 10

### Receptor Purification

Binding of biotinyl-receptor to GH₄ C1 membranes is carried out as described above. Incubations are for 1 hour at room temperature. In the standard purification protocol, the binding incubations contain 10 nM Bio-S29. ¹²⁵I ligand is added as a tracer at levels of 5,000-100,000 cpm per mg of membrane protein. Control incubations contain 10 µM cold ligand to saturate the receptor with non-biotinylated ligand.

Solubilization of receptor:ligand complex also is carried out as described above, with 0.15% deoxycholate:lysolecithin in solubilization buffer containing 0.2 mM MgCl₂, to obtain 100,000 x g supernatants containing solubilized R:L complex.

Immobilized streptavidin (streptavidin cross-linked to 6% beaded agarose, Pierce Chemical Co.; "SA-agarose") is washed in solubilization buffer and added to the solubilized membranes as 1/30 of the final volume. This mixture is incubated with constant stirring by end-over-end rotation for 4-5 hours at 4-10 °C. Then the mixture is applied to a column and the non-bound material is washed through. Binding of radioligand to SA-agarose is determined by comparing cpm in the 100,000 x g supernatant with that in the column effluent after adsorption to SA-agarose. Finally, the column is washed with 12-15 column volumes of solubilization buffer+0.15% deoxycholate:lysolecithin +1/500 (vol/vol) 100 x 4pase.

The streptavidin column is eluted with.solubilization buffer+0.1 mM EDTA+0.1 mM EGTA+0.1 mM GTP-gamma-S (Sigma)+0.15% (wt/vol) deoxycholate:lysolecithin +1/1000 (vol/vol) 100.times.4pase. First, one column volume of elution buffer is passed through the column and flow is stopped for 20-30 minutes. Then 3-4 more column volumes of elution buffer are passed through. All the eluates are pooled.

Eluates from the streptavidin column are incubated overnight (12-15 hours) with immobilized wheat germ agglutinin (WGA agarose, Vector Labs) to adsorb the receptor via interaction of covalently bound carbohydrate with the WGA lectin. The ratio (vol/vol) of WGA-agarose to streptavidin column eluate is generally 1:400. A range from 1:1000 to 1:200 also can be used. After the binding step, the resin is pelleted by centrifugation, the supernatant is removed and saved, and the resin is washed 3 times (about 2 minutes each) in buffer containing 50 mM HEPES, pH 8,5 mM MgCl₂, and 0.15% deoxycholate:lysolecithin. To elute the WGA-bound receptor, the resin is extracted three times by repeated mixing (vortex mixer on low speed) over a 15-30 minute period on ice, with 3 resin columns each time, of 10 mM N-N'-N"-triacetylchitotriose in the same HEPES buffer used to wash the resin. After each elution step, the resin is centrifuged down and the supernatant is carefully removed, free of WGA-agarose pellets. The three, pooled eluates contain the final, purified receptor. The material non-bound to WGA contain G protein subunits specifically eluted from the streptavidin column, as well as non-specific contaminants. All these fractions are stored frozen at -90°C.

### EXAMPLE 11

### Identification of a test compound which binds to a lipoxin A₄ receptor-like polypeptide

Purified lipoxin A₄ receptor-like polypeptides comprising a glutathione-S- transferase protein and absorbed onto glutathione-derivatized wells of 96-well microtiter plates are contacted with test compounds from a small molecule library at pH 7.0 in a physiological buffer solution, lipoxin A₄ receptor-like polypeptides comprise an amino acid sequence shown in SEQ ID NO. 2. The test compounds comprise a fluorescent tag. The samples are incubated for 5 minutes to one hour. Control samples are incubated in the absence of a test compound.

The buffer solution containing the test compounds is washed from the wells. Binding of a test compound to a lipoxin A₄ receptor-like polypeptide is detected by fluorescence measurements of the contents of the wells. A test compound which increases the fluorescence in a well by at least 15% relative to fluorescence of a well in which a test compound was not incubated is identified as a compound which binds to a lipoxin A₄ receptor-like polypeptide.

### EXAMPLE 12

### Identification of a test compound which decreases lipoxin A₄ receptor-like protein gene expression

A test compound is administered to a culture of CHO cells transfected with a lipoxin A₄ receptor-like polynucleotide and incubated at 37°C for 10 to 45 minutes. A culture of the same type of cells incubated for the same time without the test compound provides a negative control.

RNA is isolated from the two cultures as described in Chirgwin *et al., Biochem. 18,* 5294-99, 1979). Northern blots are prepared using 20 to 30 g total RNA and hybridized with a ³²P-labeled lipoxin A₄ receptor-like protein-specific probe at 65°C in Express-hyb (CLONTECH). The probe comprises at least 11 contiguous nucleotides selected from the complement of SEQ ID NO.1. A test compound which decreases the lipoxin A₄ receptor- like protein-specific signal relative to the signal obtained in the absence of the test compound is identified as an inhibitor of lipoxin A₄ receptor-like protein gene expression.

### EXAMPLE 13

### Treatment of inflammation with a reagent which specifically binds to a lipoxin A₄ receptor- like protein gene product

Synthesis of antisense lipoxin A₄ receptor-like protein oligonucleotides comprising at least 11 contiguous nucleotide selected from the complement of SEQ ID NO. 1 is performed on a Pharmacia Gene Assembler series synthesizer using the phosphoramidite procedure (Uhlmann *et al., Chem. Rev. 90,* 534-83, 1990). Following assembly and deprotection, oligonucleotides are ethanol-precipitated twice, dried, and suspended in phosphate-buffered saline (PBS) at the desired concentration. Purity of these oligonucleotides is tested by capillary gel electrophoreses and ion exchange HPLC. Endotoxin levels in the oligonucleotide preparation are determined using the Luminous Amebocyte Assay (Bang, *Biol. Bull. (Woods Hole, Mass.) 105,* 361-362, 1953).

The antisense oligonucleotides are injected directly into an inflamed tissue in an aqueous medium (an aqueous composition) at a concentration of 0.1-100 M with a needle. The needle is placed in the tissue and withdrawn while expressing the aqueous composition within the tissue.

The inflamed tissue is monitored over a period of days or weeks. Additional injections of the antisense oligonucleotides may be given during that time. The inflammation in the tissue gradually decreases.

### EXAMPLE 14

### Tissue-specific expression of lipoxin A4 receptor-like polypeptide

Expression profiling is based on a quantitative polymerase chain reaction (PCR) analysis, also called kinetic analysis, first described in Higuchi et al., 1992 and Higuchi et al., 1993. The principle is that at any given cycle within the exponential phase of PCR, the amount of product is proportional to the initial number of template copies. Using this technique, the expression levels of particular genes, which are transcribed from the chromosomes as messenger RNA (mRNA), are measured by first making a DNA copy (cDNA) of the mRNA, and then performing quantitative PCR on the cDNA, a method called quantitative reverse transcription-polymerase chain reaction (quantitative RT-PCR).

Quantitative RT-PCR analysis of RNA from different human tissues was performed to investigate the tissue distribution of lipoxin A₄ receptor-like polypeptide mRNA. In most cases, 25 .mu.g of total RNA from various tissues (including Human Total RNA Panel I-V, Clontech Laboratories, Palo Alto, CA, USA) was used as a template to synthsize first-strand cDNA using the SUPERSCRIPT^{™} First-Strand Synthesis System for RT-PCR (Life Technologies, Rockville , MD, USA). First-strand cDNA synthesis was carried out according to the manufacturer's protocol using oligo (dT) to hybridize to the 3' poly A tails of mRNA and prime the synthesis reaction. 10 ng of the first-strand cDNA was then used as template in a polymerase chain reaction. In other cases, 10 ng of commercially available cDNAs (Human Immune System MTC Panel and Human Blood Fractions MTC Panel, Clontech Laboratories, Palo Alto, CA, USA) were used as template in a polymerase chain reaction. The polymerase chain reaction was performed in a LightCycler (Roche Molecular Biochemicals, Indianapolis, IN, USA), in the presence of the DNA-binding fluorescent dye SYBR Green I which binds to the minor groove of the DNA double helix, produced only when double-stranded DNA is successfully synthesized in the reaction (Morrison et al., 1998). Upon binding to double-stranded DNA, SYBR Green I emits light that can be quantitatively measured by the LightCycler machine. The polymerase chain reaction was carried out using oligonucleotide primers LBRI004-L4 (TCTGTGCCCAGTCCCTGTGATGAA) and LBRI004-R2 (TCTGTCTGCCCTGGGCTCTTTCAC) and measurements of the intensity of emitted light were taken following each cycle of the reaction when the reaction had reached a temperature of 91 degrees C. Intensities of emitted light were converted into copy numbers of the gene transcript per nanogram of template cDNA by comparison with simultaneously reacted standards of known concentration.

To correct for differences in mRNA transcription levels per cell in the various tissue types, a normalization procedure was performed using similarly calculated expression levels in the various tissues of five different housekeeping genes: glyceraldehyde-3-phosphatase (G3PDH), hypoxanthine guanine phophoribosyl transferase (HPRT), beta-actin, porphobilinogen deaminase (PBGD), and beta-2-microglobulin. The level of housekeeping gene expression is considered to be relatively constant for all tissues (Adams et al., 1993, Adams et al., 1995, Liew et al., 1994) and therefore can be used as a gauge to approximate relative numbers of cells per .mu.g of total RNA used in the cDNA synthesis step. Except for the use of a slightly different set of housekeeping genes and the use of the LightCycler system to measure expression levels, the normalization procedure was essentially the same as that described in the RNA Master Blot User Manual, Apendix C (1997, Clontech Laboratories, Palo Alto, CA, USA). In brief, expression levels of the five housekeeping genes in all tissue samples were measured in three independent reactions per gene using the LightCycler and a constant amount (25 .mu.g) of starting RNA. The calculated copy numbers for each gene, derived from comparison with simultaneously reacted standards of known concentrations, were recorded and converted into a percentage of the sum of the copy numbers of the gene in all tissue samples. Then for each tissue sample, the sum of the percentage values for each gene was calculated, and a normalization factor was calculated by dividing the sum percentage value for each tissue by the sum percentage value of one of the tissues arbitrarily selected as a standard. To normalize an experimentally obtained value for the expression of a particular gene in a tissue sample, the obtained value was multiplied by the normalization factor for the tissue tested. This normalization method was used for all tissues except those derived from the Human Blood Fractions MTC Panel, which showed dramatic variation in some housekeeping genes depending on whether the tissue had been activated or not. In these tissues, normalization was carried out with a single housekeeping gene, beta-2-microglobulin.

Results are given below, showing the experimentally obtained copy numbers of mRNA per 10 ng of first-strand cDNA on the left and the normalized values on the right. RNAs used for the cDNA synthesis, along with their supplier and catalog numbers are shown in tables 1 and 2.

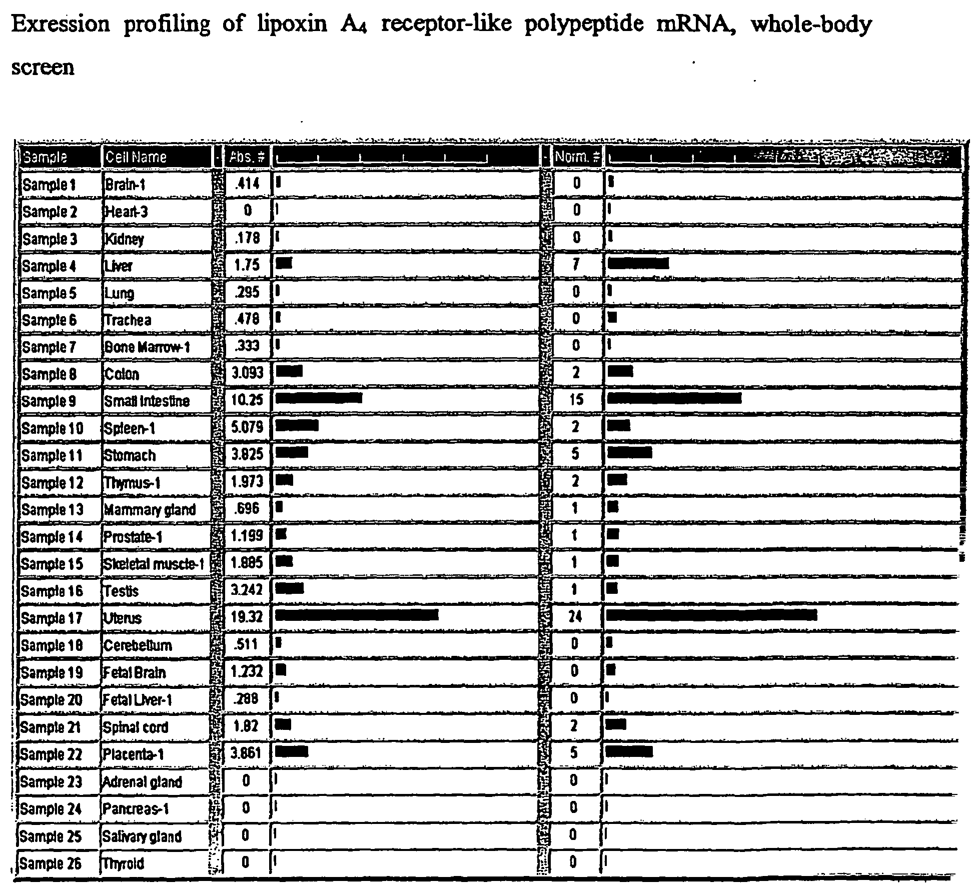

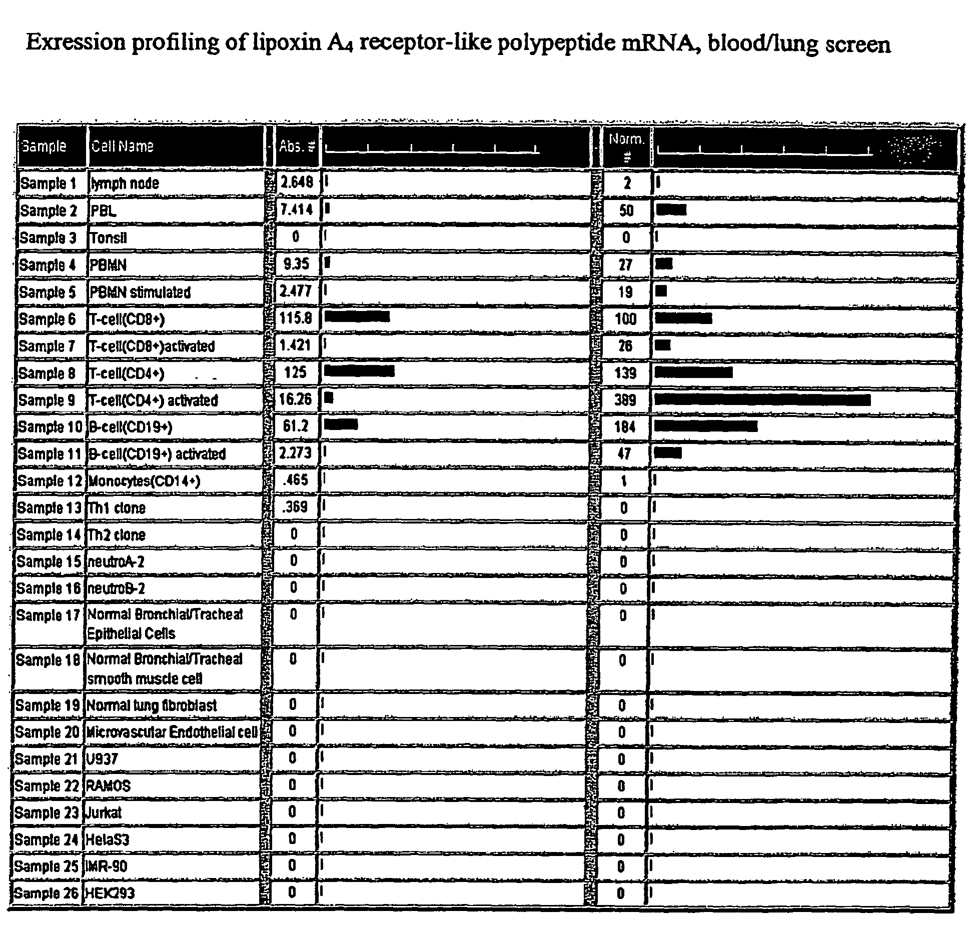

**Table 1: Whole-body-screen tissues**

| Tissue | Supplier | Panel name and catalog number |
|---|---|---|
| 1. brain | Clontech | Human Total RNA Panel I, K4000-1 |
| 2. heart | Clontech | Human Total RNA Panel I, K4000-1 |
| 3. kidney | Clontech | Human Total RNA Panel I, K4000-1 |
| 4. liver | Clontech | Human Total RNA Panel I, K4000-1 |
| 5. lung | Clontech | Human Total RNA Panel I, K4000-1 |
| 6. trachea | Clontech | Human Total RNA Panel I, K4000-1 |
| 7. bone marrow | Clontech | Human Total RNA Panel II, K4001-1 |
| 8. colon | Clontech | Human Total RNA Panel II, K4001-1 |
| 9. small intestine | Clontech | Human Total RNA Panel II, K4001-1 |
| 10. spleen | Clontech | Human Total RNA Panel II, K4001-1 |
| 11. stomach | Clontech | Human Total RNA Panel II, K4001-1 |
| 12. thymus | Clontech | Human Total RNA Panel II, K4001-1 |
| 13. mammary gland | Clontech | Human Total RNA Panel III, K4002-1 |
| 14. skeletal muscle | Clontech | Human Total RNA Panel III, K4002-1 |
| 15. prostate | Clontech | Human Total RNA Panel III, K4002-1 |
| 16. testis | Clontech | Human Total RNA Panel III, K4002-1 |
| 17. uterus | Clontech | Human Total RNA Panel III, K4002-1 |
| 18. cerebellum | Clontech | Human Total RNA Panel IV, K4003-1 |
| 19. fetal brain | Clontech | Human Total RNA Panel IV, K4003-1 |
| 20. fetal liver | Clontech | Human Total RNA Panel IV, K4003-1 |
| 21. spinal cord | Clontech | Human Total RNA Panel IV, K4003-1 |
| 22. placenta | Clontech | Human Total RNA Panel IV, K4003-1 |
| 23. adrenal gland | Clontech | Human Total RNA Panel V, K4004-1 |
| 24. pancreas | Clontech | Human Total RNA Panel V, K4004-1 |
| 25. salivary gland | Clontech | Human Total RNA Panel V, K4004-1 |
| 26. thyroid | Clontech | Human Total RNA Panel V, K4004-1 |

**Table 2: Blood/lung-screen tissues**

| Tissue | Supplier | Panel name and catalog number |
|---|---|---|
| 1. lymph node | Clontech | Human Immune System MTC Panel, K1426-1 |
| 2. peripheral blood leukocytes | Clontech | Human Immune System MTC Panel, K1426-1 |
| 3. tonsil | Clontech | Human Immune System MTC Panel, K1426-1 |
| 4. peripheral blood mononuclear cells | Clontech | Human Blood Fractions MTC Panel, K1428-1 |
| 5. peripheral blood mononuclear cells - activated | Clontech | Human Blood Fractions MTC Panel, K1428-1 |
| 6. T-cell (CD8+) | Clontech | Human Blood Fractions MTC Panel, K1428-1 |
| 7. T-cell (CD8+) - activated | Clontech | Human Blood Fractions MTC Panel, K1428-1 |
| 8. T-cell (CD4+) | Clontech | Human Blood Fractions MTC Panel, K1428-1 |
| 9. T-cell (CD4+) - activated | Clontech | Human Blood Fractions MTC Panel, K1428-1 |
| 10. B-cell (CD19+) | Clontech | Human Blood Fractions MTC Panel, K1428-1 |
| 11. B-cell (CD19+) - activated | Clontech | Human Blood Fractions MTC Panel, K1428-1 |
| 12. Monocytes (CD14+) | Clontech | Human Blood Fractions MTC Panel, K1428-1 |
| 13. Th1 clone | In-house | |
| 14. Th2 clone | In-house | |
| 15. neutrophil | In-house | |
| 16. neutrophil | In-house | |
| 17. Normal Bronchial/Tracheal Epithelial Cells | In-house | |
| 18. Normal Bronchial/Tracheal smooth muscle cell | In-house | |
| 19. Normal lung fibroblast | In-house | |
| 20. Microvascular Endothelial cell | In-house | |
| 21. U937 | In-house | |
| 22. RAMOS | In-house | |
| 23.Jurkat | In-house | |
| 24. HelaS3 | In-house | |
| 25. IMR-90 | In-house | |
| 26. HEK293 | In-house | |

In a summary lipoxin A4 receptor-like polypeptide is highly expressed in uterus, liver, placenta, and the gastrointestinal system, and lower levels of expression are found in the spleen, thymus, and spinal cord. It also exhibits high expression in peripheral blood leukocytes, where it appears to be mainly expressed by T and B cells. Mitogen activation of CD8⁺ T cells and CD19⁺ B cells appears to down-regulate the expression of lipoxin A₄ receptor-like polypeptide, whereas the opposite effect, upregulation, is seen after mitogen activation of CD4⁺ T cells.

### References

Higuchi, R., Dollinger, G., Walsh, P.S. and Griffith, R. (1992) Simultaneous amplification and detection of specific DNA sequences. *BioTechnology* 10:413-417.
Higuchi, R., Fockler, C., Dollinger, G. and Watson, R. (1993) Kinetic PCR analysis: real-time monitoring of DNA amplification reactions. *BioTechnology* 11:1026-1030.
T.B. Morrison, J.J. Weis & C.T. Wittwer .(1998) Quantification of low-copy transcripts by continuous SYBR Green I monitoring during amplification. *Biotechniques* 24**:**954-962.
Adams, M. D., Kerlavage, A. R., Fields, C. & Venter, C. (1993) 3,400 new expressed sequence tags identify diversity of transcripts in human brain. *Nature Genet.* 4:256-265.
Adams, M. D., et al. (1995) Initial assessment of human gene diversity and expression patterns based upon 83 million nucleotides of cDNA sequence. *Nature* 377 supp:3-174.
Liew, C. C., Hwang, D. M., Fung, Y. W., Laurenson, C., Cukerman, E., Tsui, S. & Lee, C. Y. (1994) A catalog of genes in the cardiovascular system as identified by expressed sequence tags. *Proc. Natl. Acad Sci. USA* 91:10145-10649.

## Claims

1. Use of a polypeptide selected from the group consisting of
(i) a polypeptide comprising an amino acid sequence which is at least 90% identical to the amino acid sequence shown in SEQ ID NO: 2;
(ii) a polypeptide comprising the amino acid sequence shown in SEQ ID NO: 2
in the screening for compounds useful in the treatment of a disease associated with inflammatory processes, wherein a test compound is contacted with the polypeptide, binding of the test compound to the polypeptide is measured and a test compound that binds to the polypeptide is selected as a compound useful in the treatment of a disease associated with inflammatory processes.

2. Use of a biological cell comprising a polynucleotide selected from the group consisting of
(i) the polynucleotide of SEQ ID NO: 1;
(ii) a polynucleotide which hybridises under stringent conditions to the polynucleotide of SEQ ID NO:1 and encodes a Lipoxin A4 Receptor-like Polypeptide
in the screening for compounds useful in the treatment of a disease associated with inflammatory processes wherein the biological cell is a T-cell and/or has been transformed with said polynucleotide, and wherein a test compound is contacted with the biological cell, the amount of mRNA transcribed or polypeptide expressed by said polynucleotide is measured and a test compound that alters the amount of mRNA transcribed or polypeptide expressed by said polynucleotide is selected as a compound useful in the treatment of a disease associated with inflammatory processes.

## Patentansprüche

1. Verwendung eines Polypeptids, das aus der aus folgenden Elementen bestehenden Gruppe ausgewählt ist:
(i) einem Polypeptid, das eine Aminosäuresequenz umfasst, die zumindest 90 % Identität mit der in Seq.-ID Nr. 2 dargestellten Aminosäuresequenz aufweist;
(ii) einem Polypeptid, das die in Seq.-ID Nr. 2 dargestellte Aminosäuresequenz umfasst,
und zwar im Screening auf Verbindungen, die bei der Behandlung einer Erkrankung, die mit Entzündungsprozessen assoziiert ist, von Nutzen sind, worin eine Testverbindung mit dem Polypeptid kontaktiert wird, die Bindung der Testverbindung an das Polypeptid gemessen wird und eine Testverbindung, die an das Polypeptid bindet, als eine bei der Behandlung einer mit Entzündungsprozessen assoziierten Erkrankung nützliche Verbindung ausgewählt wird.

2. Verwendung einer biologischen Zelle, die ein Polynucleotid umfasst, das aus der aus folgenden Elementen bestehenden Gruppe ausgewählt wurde:
(i) dem Polynucleotid aus Seq.-ID Nr. 1;
(ii) einem Polynucleotid, das unter stringenten Bedingungen an das Polynucleotid aus Seq.-ID Nr. 1 hybridisiert und für ein Lipoxin-A1-rezeptorartiges Polypeptid kodiert,
und zwar im Screening auf Verbindungen, die bei der Behandlung einer Erkrankung, die mit Entzündungsprozessen assoziiert ist, von Nutzen sind, worin die biologische Zelle eine T-Zelle ist und/oder mit dem Polynucleotid transformiert wurde und worin eine Testverbindung mit der biologischen Zelle kontaktiert wird, die Menge der transkribierten mRNA oder des von dem Polynucleotid exprimierten Polypeptids gemessen wird und eine Testverbindung, die die Menge der transkribierten mRNA oder des von dem Polynucleotid exprimierten Polypeptids verändert, als eine Verbindung ausgewählt wird, die bei der Behandlung einer mit Entzündungsprozessen assoziierten Erkrankung nützlich ist.

## Revendications

1. Utilisation d'un polypeptide choisi dans le groupe constitué par :
(i) un polypeptide comprenant une séquence d'aminoacides qui est identique au moins à 90 % à la séquence d'aminoacides représentée dans SEQ ID NO : 2 ;
(ii) un polypeptide comprenant la séquence d'aminoacides représentée dans SEQ ID NO : 2
dans le criblage de composés utiles dans le traitement d'une maladie associée à des processus inflammatoires, dans laquelle un composé à tester est mis en contact avec le polypeptide, la liaison du composé à tester au polypeptide est mesurée et un composé à tester qui se lie au polypeptide est sélectionné en tant que composé utile dans le traitement d'une maladie associée à des processus inflammatoires.

2. Utilisation d'une cellule biologique comprenant un polynucléotide choisi dans le groupe constitué par :
(i) le polynucléotide de SEQ ID NO : 1 ;
(ii) un polynucléotide qui s'hybride, dans des conditions stringentes, au polynucléotide de SEQ ID NO : 1 et code pour un Polypeptide de type Récepteur de la Lipoxine A4
dans le criblage de composés utiles dans le traitement d'une maladie associée à des processus inflammatoires, dans laquelle la cellule biologique est un lymphocyte T et/ou a été transformée avec ledit polynucléotide, et dans laquelle un composé à tester est mis en contact avec la cellule biologique, la quantité d'ARNm transcrit ou de polypeptide exprimé par ledit polynucléotide est mesurée et un composé à tester qui modifie la quantité d'ARNm transcrit ou de polypeptide exprimé par ledit polynucléotide est sélectionné en tant que composé utile dans le traitement d'une maladie associée à des processus inflammatoires.


### SEQUENCE LISTING

<110> Bayer AG
<120> REGULATION OF HUMAN LIPOXIN A4 RECEPTOR-LIKE PROTEIN
<130> REG. OF HUMAN LIPOXIN A4 RECEPTOR
<140>
   <141>
<150> 60/189,037
   <151> 2000-03-14
<160> 2
<170> PatentIn Ver. 2.0
<210> 1
   <211> 2300
   <212> DNA
   <213> Human
<400> 1
   tacatggcag aagattaagt ctgtctggac agtgtctcat gcctgtaatc tcaacatttc 60
   aggaggccaa ggtaggagga tcacttgagc tcacgagttc aagaccagcc tgggcaacac 120
   agtgagacct tgtttctact aaaaatttaa aaagtagtgg gtgcacacct gtagtcccag 180
   ctactaggga ggctgagatg ggagggtcgc tggaacccag gaggtggaag ctgcagggac 240
   tgtgccactg cactcatcct gggcaataga gcaaggccct gtctctcaaa aaaaaaaaaa 300
   agaaaagaaa agaaaagtct gggttgagcc ctggcacctc ccttcctacc ttcactgatt 360
   ctctgaacct tcctgtcctc gcctgtaaag tagattgtat gaggactcca tgaggtcatc 420
   cacttcaagt ccttggcata ggataattac tcaaaaggtg atgacaatgg cgcagggagg 480
   gatggtgact tgcctggaga tgcacagcac cgtctctccc atactcggtc attcacacca 540
   tcattgattc accaggcacc cactccgtgt ccagcaggac tctggggacc ccaaatggac 600
   actaccatgg aagctgacct gggtgccact ggccacaggc cccgcacaga gcttgatgat 660
   gaggactcct acccccaagg tggctgggac acggtcttcc tggtggccct gctgctcctt 720
   gggctgccag ccaatgggtt gatggcgtgg ctggccggct cccaggcccg gcatggagct 780
   ggcacgcgtc tggcgctgct cctgctcagc ctggccctct ctgacttctt gttcctggca 840
   gcagcggcct tccagatcct agagatccgg catgggggac actggccgct ggggacagct 900
   gcctgccgct tctactactt cctatggggc gtgtcctact cctccggcct cttcctgctg 960
   gccgccctca gcctcgaccg ctgcctgctg gcgctgtgcc cacactggta ccctgggcac 1020
   cgcccagtcc gcctgcccct ctgggtctgc gccggtgtct gggtgctggc cacactcttc 1080
   agcgtgccct ggctggtctt ccccgaggct gccgtctggt ggtacgacct ggtcatctgc 1140
   ctggacttct gggacagcga ggagctgtcg ctgaggatgc tggaggtcct ggggggcttc 1200
   ctgcctttcc tcctgctgct cgtctgccac gtgctcaccc aggccacagc ctgtcgcacc 1260
   tgccaccgcc aacagcagcc cgcagcctgc cggggcttcg cccgtgtggc caggaccatt 1320
   ctgtcagcct atgtggtcct gaggctgccc taccagctgg cccagctgct ctacctggcc 1380
   ttcctgtggg acgtctactc tggctacctg ctctgggagg ccctggtcta ctccgactac 1440
   ctgatcctac tcaacagctg cctcagcccc ttcctctgcc tcatggccag tgccgacctc 1500
   cggaccctgc tgcgctccgt gctctcgtcc ttcgcggcag ctctctgcga ggagcggccg 1560
   ggcagcttca cgcccactga gccacagacc cagctagatt ctgagggtcc aactctgcca 1620
   gagccgatgg cagaggccca gtcacagatg gatcctgtgg cccagcctca ggtgaacccc 1680
   acactccagc cacgatcgga tcccacagct cagccacagc tgaaccctac ggcccagcca 1740
   cagtcggatc ccacagccca gccacagctg aacctcatgg cccagccaca gtcagattct 1800
   gtggcccagc cacaggcaga cactaacgtc cagacccctg cacctgctgc cagttctgtg 1860
   cccagtccct gtgatgaagc ttccccaacc ccatcctcgc atcctacccc aggggccctt 1920
   gaggacccag ccacacctcc tgcctctgaa ggagaaagcc ccagcagcac cccgccagag 1980
   gcggccccgg gcgcaggccc cacgtgaggg tccaggaaca cgcaggccca ccagagcagt 2040
   gaaagagccc agggcagaca gaggaaccag ccagtcagac aggtggggag ccgccgacag 2100
   ctttgtcctt aaaaaccctg ctgagtccgt caggcctgga aggaggactt gagggagggg 2160
   aaacaatcca gccagaagtc tcaggcagtt ccatgtcagc gacccctgct cccggccatc 2220
   agccttttct gtggttgctc ccaacacaca cacagtcgcc cgacagcccc caaaccgcag 2280
   ctaatggcat cttgcggggt                                             2300
<210> 2
   <211> 470
   <212> PRT
   <213> Human
<400> 2

### Sequence Listing

<110> Bayer AG
<120> REGULATION OF HUMAN LIPOXIN A4 RECEPTOR-LIKE PROTEIN
<130> REG. OF HUMAN LIPOXIN A4 RECEPTOR
<140>
   <141>
<150> 60/189,037
   <151> 2000-03-14
<160> 2
<170> PatentIn Ver. 2.0
<210> 1
   <211> 2300
   <212> DNA
   <213> Human
<400> 1
   tacatggcag aagattaagt ctgtctggac agtgtctcat gcctgtaatc tcaacatttc 60
   aggaggccaa ggtaggagga tcacttgagc tcacgagttc aagaccagcc tgggcaacac 120
   agtgagacct tgtttctact aaaaatttaa aaagtagtgg gtgcacacct gtagtcccag 180
   ctactaggga ggctgagatg ggagggtcgc tggaacccag gaggtggaag ctgcagggac 240
   tgtgccactg cactcatcct gggcaataga gcaaggccct gtctctcaaa aaaaaaaaaa 300
   agaaaagaaa agaaaagtct gggttgagcc ctggcacctc ccttcctacc ttcactgatt 360
   ctctgaacct tcctgtcctc gcctgtaaag tagattgtat gaggactcca tgaggtcatc 420
   cacttcaagt ccttggcata ggataattac tcaaaaggtg atgacaatgg cgcagggagg 480
   gatggtgact tgcctggaga tgcacagcac cgtctctccc atactcggtc attcacacca 540
   tcattgattc accaggcacc cactccgtgt ccagcaggac tctggggacc ccaaatggac 600
   actaccatgg aagctgacct gggtgccact ggccacaggc cccgcacaga gcttgatgat 660
   gaggactcct acccccaagg tggctgggac acggtcttcc tggtggccct gctgctcctt 720
   gggctgccag ccaatgggtt gatggcgtgg ctggccggct cccaggcccg gcatggagct 780
   ggcacgcgtc tggcgctgct cctgctcagc ctggccctct ctgacttctt gttcctggca 840
   gcagcggcct tccagatcct agagatccgg catgggggac actggccgct ggggacagct 900
   gcctgccgct tctactactt cctatggggc gtgtcctact cctccggcct cttcctgctg 960
   gccgccctca gcctcgaccg ctgcctgctg gcgctgtgcc cacactggta ccctgggcac 1020
   cgcccagtcc gcctgcccct ctgggtctgc gccggtgtct gggtgctggc cacactcttc 1080
   agcgtgccct ggctggtctt ccccgaggct gccgtctggt ggtacgacct ggtcatctgc 1140
   ctggacttct gggacagcga ggagctgtcg ctgaggatgc tggaggtcct ggggggcttc 1200
   ctgcctttcc tcctgctgct cgtctgccac gtgctcaccc aggccacagc ctgtcgcacc 1260
   tgccaccgcc aacagcagcc cgcagcctgc cggggcttcg cccgtgtggc caggaccatt 1320
   ctgtcagcct atgtggtcct gaggctgccc taccagctgg cccagctgct ctacctggcc 1380
   ttcctgtggg acgtctactc tggctacctg ctctgggagg ccctggtcta ctccgactac 1440
   ctgatcctac tcaacagctg cctcagcccc ttcctctgcc tcatggccag tgccgacctc 1500
   cggaccctgc tgcgctccgt gctctcgtcc ttcgcggcag ctctctgcga ggagcggccg 1560
   ggcagcttca cgcccactga gccacagacc cagctagatt ctgagggtcc aactctgcca 1620
   gagccgatgg cagaggccca gtcacagatg gatcctgtgg cccagcctca ggtgaacccc 1680
   acactccagc cacgatcgga tcccacagct cagccacagc tgaaccctac ggcccagcca 1740
   cagtcggatc ccacagccca gccacagctg aacctcatgg cccagccaca gtcagattct 1800
   gtggcccagc cacaggcaga cactaacgtc cagacccctg cacctgctgc cagttctgtg 1860
   cccagtccct gtgatgaagc ttccccaacc ccatcctcgc atcctacccc aggggccctt 1920
   gaggacccag ccacacctcc tgcctctgaa ggagaaagcc ccagcagcac cccgccagag 1980
   gcggccccgg gcgcaggccc cacgtgaggg tccaggaaca cgcaggccca ccagagcagt 2040
   gaaagagccc agggcagaca gaggaaccag ccagtcagac aggtggggag ccgccgacag 2100
   ctttgtcctt aaaaaccctg ctgagtccgt caggcctgga aggaggactt gagggagggg 2160
   aaacaatcca gccagaagtc tcaggcagtt ccatgtcagc gacccctgct cccggccatc 2220
   agccttttct gtggttgctc ccaacacaca cacagtcgcc cgacagcccc caaaccgcag 2280
   ctaatggcat cttgcggggt                                             2300
<210> 2
   <211> 470
   <212> PRT
   <213> Human
<400> 2
   Met Asp Thr Thr Met Glu Ala Asp Leu Gly Ala Thr Gly His Arg Pro
   1               5                   10                  15
   Arg Thr Glu Leu Asp Asp Glu Asp Ser Tyr Pro Gln Gly Gly Trp Asp
               20                  25                  30
   Thr Val Phe Leu Val Ala Leu Leu Leu Leu Gly Leu Pro Ala Asn Gly
           35                  40                  45
   Leu Met Ala Trp Leu Ala Gly Ser Gln Ala Arg His Gly Ala Gly Thr
       50                  55                  60
   Arg Leu Ala Leu Leu Leu Leu Ser Leu Ala Leu Ser Asp Phe Leu Phe
   65                  70                  75                  80
   Leu Ala Ala Ala Ala Phe Gln Ile Leu Glu Ile Arg His Gly Gly His
                   85                  90                  95
   Trp Pro Leu Gly Thr Ala Ala Cys Arg Phe Tyr Tyr Phe Leu Trp Gly
               100                 105                 110
   Val Ser Tyr Ser Ser Gly Leu Phe Leu Leu Ala Ala Leu Ser Leu Asp
           115                 120                 125
   Arg Cys Leu Leu Ala Leu Cys Pro His Trp Tyr Pro Gly His Arg Pro
       130                 135                 140
   Val Arg Leu Pro Leu Trp Val Cys Ala Gly Val Trp Val Leu Ala Thr
   145                 150                 155                 160
   Leu Phe Ser Val Pro Trp Leu Val Phe Pro Glu Ala Ala Val Trp Trp
                   165                 170                 175
   Tyr Asp Leu Val Ile Cys Leu Asp Phe Trp Asp Ser Glu Glu Leu Ser
               180                 185                 190
   Leu Arg Met Leu Glu Val Leu Gly Gly Phe Leu Pro Phe Leu Leu Leu
           195                 200                 205
   Leu Val Cys His Val Leu Thr Gln Ala Thr Ala Cys Arg Thr Cys His
       210                 215                 220
   Arg Gln Gln Gln Pro Ala Ala Cys Arg Gly Phe Ala Arg Val Ala Arg
   225                 230                 235                 240
   Thr Ile Leu Ser Ala Tyr Val Val Leu Arg Leu Pro Tyr Gln Leu Ala
                   245                 250                 255
   Gln Leu Leu Tyr Leu Ala Phe Leu Trp Asp Val Tyr Ser Gly Tyr Leu
               260                 265                 270
   Leu Trp Glu Ala Leu Val Tyr Ser Asp Tyr Leu Ile Leu Leu Asn Ser
           275                 280                 285
   Cys Leu Ser Pro Phe Leu Cys Leu Met Ala Ser Ala Asp Leu Arg Thr
       290                 295                 300
   Leu Leu Arg Ser Val Leu Ser Ser Phe Ala Ala Ala Leu Cys Glu Glu
   305                 310                 315                 320
   Arg Pro Gly Ser Phe Thr Pro Thr Glu Pro Gln Thr Gln Leu Asp Ser
                   325                 330                 335
   Glu Gly Pro Thr Leu Pro Glu Pro Met Ala Glu Ala Gln Ser Gln Met
               340                 345                 350
   Asp Pro Val Ala Gln Pro Gln Val Asn Pro Thr Leu Gln Pro Arg Ser
           355                 360                 365
   Asp Pro Thr Ala Gln Pro Gln Leu Asn Pro Thr Ala Gln Pro Gln Ser
       370                 375                 380
   Asp Pro Thr Ala Gln Pro Gln Leu Asn Leu Met Ala Gln Pro Gln Ser
   385                 390                 395                 400
   Asp Ser Val Ala Gln Pro Gln Ala Asp Thr Asn Val Gln Thr Pro Ala
                   405                 410                 415
   Pro Ala Ala Ser Ser Val Pro Ser Pro Cys Asp Glu Ala Ser Pro Thr
               420                 425                 430
   Pro Ser Ser His Pro Thr Pro Gly Ala Leu Glu Asp Pro Ala Thr Pro
           435                 440                     445
   Pro Ala Ser Glu Gly Glu Ser Pro Ser Ser Thr Pro Pro Glu Ala Ala
       450                 455                     460
   Pro Gly Ala Gly Pro Thr
   465                 470